# EUROPÄISCHE PATENTSCHRIFT

(11) **EP 3 378 448 B1**
(45) Veröffentlichungstag und Bekanntmachung des Hinweises auf die Patenterteilung: **03.06.2020**
(21) Anmeldenummer: 18165038.3
(22) Anmeldetag: 16.01.2015
(51) Int. Cl.: A61F 5/01

(54) **MODULARE KNIEORTHESE**
MODULAR KNEE ORTHOTIC
ORTHÈSE DU GENOU MODULAIRE

(30) Priorität: 17.01.2014 DE 102014100527
(43) Veröffentlichungstag der Anmeldung: 26.09.2018
(62) Teilanmeldung aus: 15151495.7
(73) Patentinhaber: Zours, Peter, 44789 Bochum (DE)
(72) Erfinder: Zours, Peter, 44789 Bochum (DE)
(74) Vertreter: Patentanwälte Bals & Vogel

(56) Entgegenhaltungen:
- WO-A1-2013/040354
- US-A1- 2006 155 229
- US-A1- 2013 253 394
- US-A1- 2013 267 878

## Beschreibung

Die vorliegende Erfindung betrifft eine modular aufgebaute Orthese, insbesondere eine Knieorthese, wie in den Ansprüchen beschrieben. Es existieren zahlreiche unterschiedliche Arten von Knieorthesen, die über verschiedene Einstellungsmöglichkeiten individuell an einen Patienten anpassbar ausgestaltet sind. Jedoch trägt diese Anpassung immer nur einen konkreten Gesundheitszustand des Patienten Rechnung. Auch kann mit den bekannten Orthesen eine optimale Anpassung an anatomische Gegebenheiten des Patienten nicht gewährleistet werden. So hat bspw. eine Messstudie ergeben, dass das Verhältnis von Ober- zum Unterschenkel, nicht wie bis dato im Stand der Technik vermutet, normativ zusammenhängt, sondern dass es außerhalb dieses angenommenen normativen Zusammenhangs sehr oft große Abweichungen gibt. Diese Abweichungen kommen bspw. durch Mehrbelastung des Oberschenkels relativ zum Unterschenkel zustande, wie dies bspw. bei Sportlern bekannt ist. Allerdings werden die bisher gängigen Knieorthesen in einer vorkonfektionierten Form mit einem normativ gleichen Größenverhältnis zwischen Ober- und Unterschenkel geliefert. Dies führt bei der Anpassung der Orthese regelmäßig zu Passproblemen, mit der Folge, dass in der Orthopädietechnik entweder die Ober- oder die Unterschenkelspange einer vorkonfektionierten Orthese zum Anpassen verbogen werden muss. Dies führt nachteilhaft dazu, dass die bei den vorkonfektionierten Orthesen eingestellte Parallelität der seitlichen Führungsgelenke unumgänglich verändert wird, was die therapeutische Funktion der Orthese deutlich negativ verfälscht. Zudem kann es durch das Verbiegen der Ober- oder Unterschenkelspange ggf. zu einem unrunden Lauf des Gelenks führen, wodurch ein erheblich höherer Verschleiß des Gelenks erfolgt.

Die Aufgabe der vorliegenden Erfindung ist es, eine Orthese, insbesondere eine Knieorthese, bereitzustellen, um diese an die anatomischen Gegebenheiten des Patienten anpassen zu können. Diese Orthese, insbesondere diese Knieorthese soll zur Auf- oder Abschulung verwendbar sein.

Die vorliegende Aufgabe wird durch eine modular aufgebaute Orthese mit den Merkmalen des Anspruchs 1 gelöst. Weitere Vorteile, Merkmale und Details der Offenbarung ergeben sich aus den Ausführungsformen in den Unteransprüchen, der Beschreibung und den Zeichnungen. Dabei gelten Merkmale und Details, die im Zusammenhang mit der offenbarten modular aufgebauten Orthese beschrieben sind, selbstverständlich auch im Zusammenhang mit den für die modular aufgebaute Orthese beschriebenen Komponenten, insbesondere für das Halteteil, die Spange und die Schiene, sowie für das Gelenk und jeweils umgekehrt, so dass bezüglich der Offenbarung zu den einzelnen Aspekten stets wechselseitig Bezug genommen wird bzw. werden kann.

Die offenbarte modular aufgebaute Orthese, insbesondere eine Knieorthese, umfassend folgende mechanisch zusammenfügbare Komponenten:
- mindestens ein Orthesengelenk,
- ein erstes Halteteil,
- ein zweites Halteteil,
- mindestens ein Verbindungselement,
- mindestens eine Schiene,
wobei die Halteteile jeweils über ein Verbindungselement an oder auf jeweils einer Schiene befestigbar sind, und wobei wenigstens zwei Schienen das erste und das zweite Halteteil mit dem Orthesengelenk verbinden, schließt die technische Lehre ein, dass das Verbindungselement zumindest ein flexibles Mittelteil aufweist und/oder gelenkig einstellbar und/oder dass die Schiene gelenkig einstellbar ist.

Durch die gelenkige Einstellung ist ein Einstellmechanismus ausgebildet, beispielsweise durch ein Gelenk, insbesondere ein Scharniergelenk, oder ein flexibles Mittelteil, welche eine Winkeleinstellung des Verbindungselementes und/oder der Schiene und somit auch eine Winkeleinstellung der mit dem Verbindungselement und/oder der Schiene verbundenen Komponenten der Orthese erlauben, so dass während des Therapieverlaufes zu jedem Zeitpunkt eine Parallelität der Gelenkschienen eingestellt werden kann. Daher kann über das Verbindungselement und/oder die Schiene die Orthese an jede spezielle Anatomie eines Patienten, insbesondere an die Anatomie des Knies, angepasst und auf spezielle Indikationen eingestellt werden. Dadurch ist es erstmalig möglich eine modulare Knieorthese mit einstellbarer Passform komplett aus Kunststoff herzustellen, da auf Grund der Einstellmöglichkeiten durch das Gelenk oder das flexible Mittelteil das bei den bekannten Knieorthesen erforderliche aber ungenaue Anschränken oder Verbiegen der Metallkomponenten nicht länger erforderlich ist. Dadurch kann insgesamt das Gewicht der modularen Orthese, insbesondere einer modularen Knieorthese, reduziert und auch deren Herstellungskosten verringert werden.

Besonders bevorzugt eignet sich für bspw. den Zweck der offenbarte Orthese als OA-Orthese ein ein- oder mehrteiliges Scharnier, welches eine individuelle Einstellbarkeit des Verbindungselements erlaubt. Als OA-Orthese wird eine zur Entlastung des medialen Knie-Kompartiments ausgestaltete Orthese verstanden. Über das bewegliche Verbindungselement kann so in vorteilhafter Weise zwischen der Ausnehmung, die zur Verbindung der Halteteile, nämlich insbesondere der Spangen mit dem Verbindungselement dient und der Aufnahme, die zur Verbindung des Verbindungselements mit der Schiene dient, im Bereich zwischen der Ausnehmung und der Aufnahme ein vordefinierter Winkel eingestellt werden. Über diese Möglichkeit der Voreinstellung über das gelenkige Verbindungselement, kann so eine vordefinierte Schieneneinstellung der Orthese, insbesondere für dessen Anwendung als OA-Orthese realisiert werden.

Insbesondere zur Verwendung der Schiene als OA-Schiene kann diese wenigstens ein Scharnier umfassen, welches bspw. als ein- oder mehrteiliges Gelenk ausgestaltet ist. In bevorzugter Weise ist dabei die Schiene über das Gelenk bzw. die Gelenke arretierbar, d. h. fest einstellbar. Eine Arretierung kann bspw. mittels verschiedener Werkzeuge, wie bspw. Schraubenzieher, Innensechskantschlüssel, Zangen etc. oder durch ein integriertes Dreh- oder Einhaksystem erzielt werden.

Das von dem Verbindungselement und/oder der Schiene umfasste Gelenk ist vorteilhaft ein Scharniergelenk, das in das Verbindungselement zwischen der Gelenkschiene und dem Halteteil oder das zwischen zwei Schienenteilen an- oder eingesetzt ist. Vorzugsweise ist das Scharniergelenk einstellbar, wobei vorteilhaft die Einstellung stufenlos erfolgt und das Scharniergelenk in der gewählten Einstellung arretiert werden kann. Die Einstellung kann aber auch über an die Winkelstellung angepasste Raststufen erfolgen. Die Einstellung des Scharniergelenkes kann vorteilhaft händisch, d. h. ohne Werkzeug, oder aber auch mittels eines Werkzeuges, wie beispielsweise einem Schraubendreher oder einem Inbusschlüssel erfolgen. Händisch kann die Einstellung des Scharniergelenkes beispielsweise mittels einer Rändelschraube oder -mutter, einem Rändelrad oder beispielsweise einer Kurbel oder einem Schieber erfolgen, welche mit dem Scharniergelenk mechanisch wirkverbunden sind. Vorteilhaft für die Einstellung des Scharniergelenkes ist dabei dessen Verdrehbarkeit.

Vorzugsweise ist das Scharniergelenk aus einem Material oder einer Materialkombination wie Kunststoff, Metall, Metalllegierungen, oder Kunststoffmetalllegierungen ausgestaltet.

Anstelle oder zusätzlich kann das Scharniergelenk auch durch ein flexibles und/oder elastisches Mittelteil des Verbindungselementes ersetzt oder ergänzt werden. Um das Verbindungselement und/oder die Schiene in einer eingestellten Winkelstellung zu fixieren, kann beispielsweise von außen ein Fixier-Clip aufgesetzt werden, der in unterschiedlichsten Varianten und Formen an die eingestellten Winkelstellungen des Verbindungselementes angepasst ist, oder durch den die Winkelstellung eingestellt wird.

Die Fixierung des Verbindungselementes und/oder der Schiene in dem eingestellten Winkel kann auch durch eine Metall- oder vorzugsweise Kunststoffspange erfolgen, die das Verbindungselement oder die Schiene zumindest abschnittsweise überspannt oder seitlich an das Verbindungselement oder die Schiene an- oder eingesetzt wird, um das Verbindungselement oder die Schiene in dem gewünschten eingestellten Winkel zu halten oder den Winkel vorzugeben.

Bei einer integralen, d. h. einer einstückigen oder monolithischen Ausgestaltung des Verbindungselementes mit dem Halteteil und/oder der Schiene kann das Scharniergelenk zwischen dem Halteteil und der Schiene eingesetzt werden, und das Halteteil und die Schiene über die damit integral ausgestalteten Verbindungselemente miteinander verbinden. Anstelle eines Fixier-Clips kann der gewünschte Winkel auch durch einen seitlich auf das Verbindungselement oder die Schiene auf- oder einzusetzenden Stabilisator in Form einer Kunststoff- oder Metallplatte o. ä. gehalten werden.

Die für das Verbindungselement aufgeführten Einstellmöglichkeiten über ein oder mehrere Scharniergelenke oder über zumindest ein flexibles Mittelteil mit deren vorteilhaften Ausgestaltungen können auch in den Schienen oder den Schienenteilen der Gelenkschiene oder in dem Gelenk, das die Schienenteile der Gelenkschiene gelenkig miteinander verbindet, ausgebildet sein, um die Funktion einer über einen Einstellmechanismus Knieorthese, insbesondere eine OA-Knieorthese, zu erzielen.

Eine modular aufgebaute Orthese im Sinne der vorliegenden Offenbarung kann eine jedwede Gelenkorthese sein, die zwei über ein Gelenk verbundene Körperteile stabilisierend unterstützt.

Entsprechend kann die vorliegende Offenbarung auch nur eine modular aufgebaute Orthese umfassen und kann auch nur auf die modular aufgebaute Orthese gerichtet werden mit den in der Beschreibung, der Figurenbeschreibung und den in den Figuren dargestellten vorteilhaften Ausführungen und mit den in der Beschreibung, der Figurenbeschreibung und den in den Figuren dargestellten vorteilhaften Ausführungen der Komponenten.

Die Halteteile sind bei der Orthese jeweils über ein Verbindungselement an oder auf jeweils einer Schiene befestigbar. Vorteilhafter Weise verbinden dabei wenigstens zwei Schienen das erste und das zweite Halteteil mit dem Orthesengelenk.

Entsprechend kann die modular aufgebaute Orthese als Knieorthese oder bspw. auch als Ellenbogengelenk- oder Knöchelgelenkorthese verstanden werden. Daher soll hier die technische Lehre der in der vorliegenden Erfindung beispielhaft dargestellten Knieorthese nicht einschränkend verstanden werden. Bei der weiteren nicht einschränkenden Darstellung einer modular aufgebauten Orthese gemäß der vorliegenden Offenbarung, wird diese am Beispiel einer Rahmen-Knieorthese beschrieben, wobei über eine Ober- bzw. Unterschenkelspange die Rahmen-Knieorthese an einem Patienten angelegt wird.

Die vorliegende Offenbarung umfasst auch nur eine Schiene und kann auch nur auf die Schiene gerichtet werden mit den in der Beschreibung, der Figurenbeschreibung und den in den Figuren dargestellten vorteilhaften Ausführungen.

Als Schiene bzw. Schienen sollen im Sinne der Offenbarung auch Schienenteile oder Schienenschenkel verstanden werden. Dabei bilden beispielsweise zwei über das Orthesengelenk verbundene Schienenschenkel bzw. Schienenteile die das Gelenk stabilisierende Schiene bzw. die das Gelenk stabilisierenden Schienen.

Gemäß der vorliegenden Offenbarung werden Halteteile, wobei es sich hier beispielhaft um eine Ober- bzw. Unterschenkelspange handelt, jeweils über das Verbindungselement an oder auf jeweils einer Schiene befestigt. Wenigstens zwei Schienen dienen dazu, um das erste Halteteil, bspw. die Oberschenkelspange, und das zweite Halteteile, bspw. die Unterschenkelspange, über die Schiene mit dem Orthesengelenk zu verbinden.

Sowohl die Halteteile als auch die Schienen und die Verbindungselemente (wie auch das weiter unten genannte Aufnahmeteil) sind als einzelne Komponenten zu verstehen, die dem Einsatz der Knieorthese angepasst aus einem vorgehaltenen Sortiment ähnlich eines Baukastensystems ausgewählt werden können.

Diese Lösung bietet den Vorteil, dass es durch die Variabilität der einzelnen Komponenten der modular aufgebauten Orthese insbesondere möglich ist, die äußere und/oder innere Kniegelenkschiene unterschiedlich hoch einzustellen, wodurch eine individuelle Varus-/Valgus-Einstellung ermöglicht wird. Diese genauen Anpassungen sind bspw. bei X- und O-Bein-Stellungen notwendig. Zudem ist es durch die Variabilität und Auswahlmöglichkeiten der einzelnen Komponenten möglich bei bspw. besonders großen bzw. besonders kleinen Patienten die Höhe der Orthese durch beidseitige Höhenverstellung den individuellen Maßen anzupassen. Im Gegensatz dazu bieten die handelsüblichen Orthesen eine vorgegebene Höhe. Das Verbindungselement, welches die Halteteile, also die Ober- bzw. Unterschenkelspange mit der Schiene verbindet ist dabei vorzugsweise starr ausgebildet.

Die vorliegende Offenbarung umfasst auch nur ein Aufnahmeelement und kann auch nur auf das Aufnahmeelement gerichtet werden mit den in der Beschreibung, der Figurenbeschreibung und den in den Figuren dargestellten vorteilhaften Ausführungen. Vorzugsweise weist die Orthese zusätzlich Aufnahmeelemente auf, insbesondere Gurtaufnahmeelemente, die es als zusätzliche Komponente der offenbarten Orthese erlauben, neben bspw. der Ober- bzw. Unterschenkelspange Gurte, die zur weiteren Stabilisierung der Orthese an dem Patienten dienen, aufzunehmen. Vorzugsweise können die Aufnahmeelemente mit den Schienen und/oder den Verbindungselementen, welche die Halteteile mit der Schiene verbinden, form- und/oder kraftschlüssig verbunden sein. Wie auch für die anderen Komponenten der offenbarten modularen Orthese geltend, können die Aufnahmeelemente unterschiedliche Größen und Materialeigenschaften aufweisen, um diese der Indikation und/oder der individuellen Anatomie beispielsweise der Beine eines Patienten angepasst als Komponente für die modular aufgebaute Orthese anbieten zu können.

Die vorliegende Offenbarung umfasst auch nur ein Halteteil und kann auch nur auf das Halteteil gerichtet werden mit den in der Beschreibung, der Figurenbeschreibung und den in den Figuren dargestellten vorteilhaften Ausführungen.

Im Sinne der vorliegenden Offenbarung soll als Halteteil eine Spange oder ein Gurt verstanden werden. Dadurch wird auch die Möglichkeit eingeschlossen, neben einer Spange auch einen Gurt über das Verbindungselement mit einer Schiene einer Orthese zu verbinden. Entsprechend ist ein Halteteil, welches als Gurt ausgestaltet ist, neben den Ober- und Unterschenkelspangen eine weitere Komponente eines Baukastenmodells der modular aufgebauten Orthese. Durch die Möglichkeit der Befestigung der als Gurt ausgestalteten Halteteile an den Aufnahmeelementen wird die Funktion der modular aufgebauten Orthese, die durch eine Ober- und Unterschenkelspange als Hartrahmen-Orthese funktioniert, durch die Funktion einer Gelenkführungsorthese ohne oder mit Extensions-/Flexionsbegrenzung sowie ggf. nach dem Vierpunktprinzip, nämlich durch Austausch der Spangen gegen die Gurte erweitert. Die modular aufgebaute Orthese im Sinne der vorliegenden Offenbarung erlaubt es auch, diese mit einseitiger Schiene zur Entlastung bzw. zur medialen/lateralen Kompartmententlastung nach dem 3-Punkt-Prinzip ohne oder mit Flexions-/Extensionsbegrenzung einzusetzen, so z. B. als Gonarthroseorthese.

Darüber hinaus ist die modular aufgebaute Orthese vorteilhaft an sämtliche speziellen Indikationen (z. B. zur Stabilisierung eines Kniegelenks mit einem vorderen oder hinteren Kreuzbandabriss) bestmöglich anpassbar, da die einzelnen Komponenten aus einem Baukastensystem frei wählbar sind. So ist es bspw. auch möglich durch ein seitenverkehrtes Einsetzen der Ober- und/oder Unterschenkelspangen oder durch die Umkehrung des Verbindungselements, welches die Halteteile, also die Ober- und/oder Unterschenkelspange, bzw. die Gurte mit der Schiene verbindet, die Spangen bzw. Gurte auf der jeweils gegenüberliegenden Beinseite zu befestigen. Dies ermöglicht, wie bereits beschrieben, die Versorgung eines Patienten mit der modular aufgebauten Orthese an dessen spezielle Indikation.

Bei anderen speziellen Indikationen ist es natürlich auch möglich, alternativ zu einer Ober- und/oder Unterschenkelspange die obere oder die untere Spange bzw. die hintere oder die vordere Spange durch ein Gurtsystem, nämlich über als Halteteile ausgestaltete Gurte zu ersetzen.

In vorteilhafterweise sind die einzelnen Komponenten der Orthese aus zumindest einem Textil, Kunststoff, Metall, Metalllegierungen und/oder einer Kombination aus mindestens zwei der Materialien Textil, Kunststoff, Metall und/oder Metalllegierungen herstellbar. Entsprechend der Beanspruchung der einzelnen Komponenten und entsprechend dem Hygienestandard können die Materialien der einzelnen Komponenten frei ausgewählt werden. Vorzugsweise bietet es sich dabei an, die Materialien so auszuwählen, dass zum einen ein hoher Tragekomfort der modularen Orthese gewährleistet ist, und zum anderen die einzelnen Komponenten bei Benutzung der modularen Orthese verschleißfrei und ohne Materialermüdung zusammenwirken. Dies ermöglicht beispielsweise die Rücknahme der einzelnen Komponenten und dessen Wiederverwertung.

Natürlich kann zur Reduzierung der Komponenten der modular aufgebauten Orthese das Verbindungselement, welches das Halteteil mit der Schiene verbindet, integral mit dem Halteteil und/oder der Schiene ausgestaltet sein. Eine integrale Ausgestaltung im Sinne der vorliegenden Offenbarung bedeutet, dass zwei miteinander verbundene Komponenten entweder einstückig/einteilig ausgestaltet sind, oder diese in einem bereits vormontierten Zustand als ein Bauteil für die modulare Orthese vorgehalten werden.

Vorzugsweise ist das Halteteil, welches als Komponente für die modular aufgebaute Orthese dient, als Spange und/oder Gurt ausgestaltet. Die Spange kann dabei aus Kunststoff oder Metall oder aus einer Kunststoffmetalllegierung und/oder Metalllegierungen herstellbar sein und der Gurt ist vorzugsweise aus einem Textil bzw. einem Kunststofftextilverbund herstellbar. Um den speziellen Indikationen gerecht zu werden, werden die als Spange ausgestalteten Halteteile in unterschiedlichen Weiten (Größen) für unterschiedliche Beinumfänge zur Verfügung gestellt. Zur Feinjustierung bzw. zur weiteren Einstellung der Spangen bzw. des Gurtes dienen zum Justieren und zur Weitenverstellung Ausnehmungen. Wird demnach bspw. eine an die Größe des Ober- bzw. Unterschenkels angepasste Spange mit dem Verbindungselement verbunden, kann über Ausnehmungen in der Spange, die bspw. als Langlöcher ausgestaltet sind, die Lage der Spange in dem Verbindungselement bzw. an dem Verbindungselement feinjustiert werden, bevor die Spange mit dem Verbindungselement bspw. über Befestigungselemente fest verbunden wird. In vorteilhafter Weise werden dabei die Befestigungselemente durch das Verbindungselement und durch die Ausnehmungen der Spange geführt, so dass die Ausnehmungen relativ zu dem Befestigungselement bewegbar sind.

Vorzugsweise eignen sich die Spange und/oder der Gurt dazu, diese den anatomischen Gegebenheiten eines Patienten (z. B. aufgrund von Muskelverschmächtigungen, die durch physiotherapeutische Maßnahmen wieder aufgebaut werden), bzw. dessen Anwendung und/oder dessen Lage angepasst auszugestalten. So ist es bspw. notwendig, z. B. die Spange an ein linkes oder ein rechtes Bein anzupassen, welche in ihrer Anatomie unterschiedlich sein können. Auch der Einsatz der Spange als vordere oder hintere Spange erfordert eine spezielle Ausgestaltung der Spange, bspw. spezielle Materialeigenschaften oder Formen der Spange.

Vorzugsweise kann die als Halteteil ausgebildete Spange auch als mehrteilige Spange als Komponente für die modular aufgebaute Orthese angeboten werden. So kann bspw. die Spange zwei- oder mehrteilig ausgestaltet sein, mit vorzugsweisen Verstellmöglichkeiten deren Umfangs bspw. über Löcher oder Langlöcher oder auch über einen Klettverschluss, wie beispielsweise Haken- und Flauschband (Filzband), Pilzkopfband und Veloursband Knöpfe, Pilzkopfband und Flauschband, Pilzkopfband auf Pilzkopfband oder extrudierte Haken/Pilze auf Wirkware. Natürlich ist es auch denkbar, die Teile der Spange anderweitig kraft- und/oder formschlüssig miteinander zu verbinden, bspw. durch Verkleben, Vernieten, Verlöten etc..

Es ist auch denkbar, das Halteteil, nämlich die Spange integral mit wenigstens einem Verbindungselement auszugestalten, über welches die Spange an oder auf einer Schiene befestigt wird.

Die als Halteteil ausgestaltete Spange ist vorzugsweise als geteilte halbe Spange zum beidseitigen Einsetzen in Ausnehmungen des Verbindungselements ausgestaltet. Dadurch eignet sich das Halteteil zum Einsetzen in das rechte oder linke Ober- und/oder Unterschenkelverbindungselement oder auch als seitliche ganze Spange zum Anfügen bzw. zum Befestigen an das rechte bzw. linke Ober- und/oder Unterschenkelverbindungselement für bspw. eine Orthese mit nur einer Schiene. Das Anfügen bzw. Befestigen der Spange kann bspw. durch Schrauben, Kletten oder Kleben oder anderweitig kraft- und/oder formschlüssig erfolgen. Durch diese Ausgestaltung des Halteteils, nämlich als halbe Spange wird der Anpressdruck am Ober- und/oder Unterschenkel vorteilhaft seitlich am Bein ober- und unterhalb der Schiene verteilt. Um die halbe Spange an dem Ober- bzw. Unterschenkel zu halten, weist diese vorteilhaft zur kraft- und/oder formschlüssigen Verbindung mit einem Gurt an dessen äußeren Ende eine Befestigungsmöglichkeit für den Gurt auf. Dabei handelt es sich vorteilhaft um ein Aufnahmeelement zur kraft- und oder formschlüssigen Verbindung mit dem Gurt. Es sind aber auch andere Befestigungselemente, wie bspw. aufsetzbare Schnallen, Schnellverschlüsse, Klettverschlüsse, wie beispielsweise Haken- und Flauschband (Filzband), Pilzkopfband und Veloursband Knöpfe, Pilzkopfband und Flauschband, Pilzkopfband auf Pilzkopfband oder extrudierte Haken/Pilze auf Wirkware, eine Haken-Ösen-Verbindung oder andere Befestigungsmöglichkeiten, die eine kraft- und/oder formschlüssige Befestigung des Gurtes mit dem Verbindungselement erlauben, denkbar, so dass es beispielsweise möglich ist, mit dem Gurt die nicht von der Spange umfasste Beinseite zu umschließen.

Vorzugsweise handelt es sich bei dem Befestigungselement, welches die halbe Spange mit einem Gurt verbindet, um ein Aufnahmeelement, welches eine Schnalle umfasst, die eine Spange des Gurtes aufnimmt, wobei die Schnalle insbesondere Teil eines Schnellverschlusses ist. Der so ausgestaltete Schnellverschluss, z. B. nach dem Prinzip ähnlich dem eines Skistiefels, dient zweckmäßig zum schnellen Öffnen und Schließen der Orthese beispielsweise bei einer Arztvisite. Zudem lässt sich über den Schnellverschluss die Anlage des Gurtes am Patienten nachjustieren.

Hinsichtlich der Formgebung, d. h. der Breite, dem Umfang und den Materialeigenschaften der Spange, sind keine Grenzen gesetzt. Zudem ist es vorteilhaft möglich, die Spange von bspw. einem Orthopädietechniker vor Einsatz in die Orthese auf Maß zuzuschneiden, bzw. diese auf eine Überlänge zu verlängern und ggf. mit einer Polsterung zu versehen.

Das Verbindungselement, welches vorzugsweise als Komponente für die modular aufgebaute Orthese einsetzbar ist, weist in vorteilhafter Weise Befestigungsmöglichkeiten zur kraft- und/oder formschlüssigen Befestigung von Halteteilen, wie bspw. einer Spange oder eines Gurtes auf. So kann bspw. mit dem Verbindungselement eine Ober- und/oder Unterschenkelspange bspw. durch Einstecken, Anschrauben, Befestigung über ein Pilzkopfstecksystem, Vernieten, Ankletten über Klettverschlüsse, wie beispielsweise Haken- und Flauschband (Filzband), Pilzkopfband und Veloursband Knöpfe, Pilzkopfband und Flauschband, Pilzkopfband auf Pilzkopfband oder extrudierte Haken/Pilze auf Wirkware, Ankleben oder sonstige Befestigungsmöglichkeiten, verbunden werden.

In vorteilhafter Weise weist das Verbindungselement eine Ausnehmung auf, die ein- oder beidseitig des Verbindungselements ausgestaltet sein kann, so dass z. B. die als Halteteil ausgestaltete Spange durch das Verbindungselement hindurch geschoben werden kann. Über die in dem Halteteil beispielsweise als Langlöcher ausgestalteten Ausnehmungen kann so über die Befestigungselemente und durch die Einführung des Halteteils über die Ausnehmungen in das Verbindungselement zusätzlich eine Feinjustierung des Halteteils erfolgen.

So kann vorzugsweise die Höhe der formschlüssigen Verbindung zwischen dem Halteteil und der Schiene variabel beim Befestigen des durch die Ausnehmungen des Verbindungselements geführten Halteteils an dem Verbindungselement, nämlich über die Ausnehmungen, die im Halteteil ausgestaltet sind, eingestellt werden.

Optional können in dem Verbindungselement Vertiefungen vorgesehen sein, in die an den Spangen angebrachte Nasen beim Einschieben einrasten, so dass die Ausnehmungen in der Spange, also in dem Halteteil mit den Befestigungselementen des Verbindungselements auf einer Linie liegen, und so eine einfache Befestigung des Halteteils mit dem Verbindungselement erlauben. Auch kann durch die Nasen, die beim Einschieben der Spange in das Verbindungselement, nämlich in den Ausnehmungen des Verbindungselements einrasten, zusätzlich zu den Befestigungselementen ein Herausrutschen der Spange verhindert werden. Alternativ kann dieser Effekt auch durch andere Verrastungsmöglichkeiten oder wellenförmige Vertiefungen erzielt werden. Zudem kann zusätzlich durch eine Arretierung bspw. einen Splint vermieden werden, dass das Halteteil, nämlich die Spange zu viel Spiel in der Ausnehmung des Verbindungselements hat, wodurch ein Wackeln der Spange vermieden werden kann.

In vorteilhafter Weise sind die Breite und Stärke der Aufnahmen in dem Verbindungselement der Breite und Stärke der Schienen angepasst, so dass Schienen unterschiedlicher Breite und Stärke an das Halteteil nämlich über das Verbindungselement adaptierbar sind.

Darüber hinaus weist das Verbindungselement vorteilhaft an die Breite oder Stärke der Halteteile angepasst Ausnehmungen auf, wodurch unterschiedliche Halteteile bspw. unterschiedlich breite oder starke Spangen an die Schienen adaptierbar sind.

Darüber hinaus weist das Verbindungselement neben den Aufnahmen für die seitlich geführten Schienen der Orthese vorteilhafter Weise zusätzliche Aufnahmen auf, die bspw. zur Befestigung von Kniegelenkschienen dienen.

Darüber hinaus umfasst das Verbindungselement vorzugsweise Befestigungselemente zum Befestigen von Aufnahmeelementen, die dazu dienen weitere Halteteile, nämlich bspw. Gurte über das Verbindungselement an der Schiene zu befestigen.

Die Befestigung der Halteteile an dem Verbindungselement erfolgt in vorteilhafter Weise zur Einstellung verschiedener Höhen bspw. über mehrere Befestigungslöcher oder Langlöcher, was insbesondere wichtig ist für die Einstellbarkeit einer OA-Orthese. Ganz allgemein lassen sich so unterschiedlich hohe Beine und somit unterschiedlich hohe Orthesen bzw. Varus-Valgus-Einstellungen durch die Höhenvariabilität über die Aufnahme- und die Befestigungselemente realisieren.

Die Form des Verbindungselements kann in vorteilhafter Weise an die Form der damit zu verbindenden Schiene und des damit zu verbindenden Halteteils angepasst sein. In vorteilhafter Weise ist das Verbindungselement rechteckig ausgestaltet. Das Verbindungselement kann aber auch bspw. in Form eines T-Stücks oder eines L-Stücks ausgestaltet sein, wobei der Teil des T-Stücks bzw. des L-Stücks, welcher zur Befestigung des Halteteils dient, abschnittsweise die Funktion des Halteteils mit übernimmt oder bzw. an die Anatomie des Patienten, d. h. bspw. in seinem Radius an den Ober- bzw. Unterschenkel angepasst ist. Zudem erweitert sich über das T-Stück bzw. L-Stück die Einstellmöglichkeit des bspw. als Spange ausgestalteten Halteteils relativ zu dem Verbindungselement. Zudem bietet es sich an, das Verbindungselement so auszugestalten, nämlich bspw. eventuell rund oder abgerundet auszugestalten, so dass der Tragekomfort durch diese Ausgestaltung des Verbindungselements erhöht wird.

Die Befestigungselemente zur Befestigung von den Aufnahmeelementen, insbesondere von den Gurtaufnahmeelementen umfassen Bohrungen mit Innengewinde und die dazugehörigen Schrauben, wobei die Schrauben bspw. als Pilzkopfschrauben ausgestaltet sein können. Bei den Befestigungselementen kann es sich aber natürlich auch um Klettverschlüsse, wie beispielsweise Haken- und Flauschband (Filzband), Pilzkopfband und Veloursband Knöpfe, Pilzkopfband und Flauschband, Pilzkopfband auf Pilzkopfband oder extrudierte Haken/Pilze auf Wirkware, Haken-Ösen-Verbindungen, Niet- bzw. Klebverbindungen, also insgesamt um Befestigungen handeln, die eine kraft- und/oder formschlüssige Befestigung der Aufnahmeelemente an den Verbindungselementen erlaubt.

Vorteilhafterweise eignet sich das Verbindungselement auch in abgeänderter Form dazu, dass bei Orthesen mit einseitigen Schienen auf der gegenüberliegenden Seite eine einfache Gelenkschiene oder z. B. ein Kunststoffteil eingesetzt werden kann, welches über die Aufnahme mit dem Verbindungselement verbunden wird.

Zudem eignet sich das Verbindungselement auch dazu, Orthesen mit einseitigen Schienen auszugestalten, die eine breitere und/oder stärkere Schiene benötigen, um eine entsprechende Stabilität der Orthese zu erlangen.

Vorzugsweise eignen sich die Schienen, die über die Aufnahme des Verbindungselements mit dem Verbindungselement verbunden sind, zum Einführen in eine Schienentasche einer Bandage. In vorteilhafter Weise dienen die Schienen dann zur Verlängerung entweder von kurzen Schienen in längeren Bandagen und oder zur Verbindung einer verlängerten Schiene mit einer außen über der Bandage getragenen Ober- und Unterschenkelspange. Zur Befestigung der Ober- und/oder Unterschenkelspange, nämlich zur Befestigung des Halteteils wird dieses vorzugsweise über eine Öffnung in der Schienentasche der Bandage in die Ausnehmung des Verbindungselements geführt und daran befestigt. Das bedeutet in vorteilhafter Weise, dass auch das Verbindungselement, welches das Halteteil, nämlich hier insbesondere die Spange mit der Schiene verbindet in die Schienentasche der Bandage einführbar ist.

In vorteilhafter Weise werden die für eine Bandage, hier bspw. eine Kniebandage, benötigten Gurte entweder an den Aufnahmeelementen an den Verbindungselementen befestigt, oder die Gurte werden außerhalb der in den Schienentaschen eingeführten Orthesenkomponenten an der Kniebandage selber befestigt, nämlich bspw. durch Klettverschlüsse, wie beispielsweise Haken- und Flauschband (Filzband), Pilzkopfband und Veloursband Knöpfe, Pilzkopfband und Flauschband, Pilzkopfband auf Pilzkopfband oder extrudierte Haken/Pilze auf Wirkware, Haken-Ösen-Verbindungen, Druckknöpfe oder Schlaufen oder eine andere kraftschlüssige Befestigung, so dass diese abgenommen werden können, um bspw. verschiedene Gurtsysteme und eventuell ebenfalls abnehmbare Gegenhaltesysteme (z. B. Gurtschnallen) einzusetzen. Dabei werden die Gurte wie auch die Gegenhaltemittel im Bereich der Befestigung an der Schienentasche (z. B. im Bereich der Klettverschlüsse oder Haken-Ösen-Verbindung) und eventuell etwas darüber hinaus, vorzugsweise aus elastischem Material gefertigt, um daran elastische und vorteilhafter Weise unelastische Gurte anzunähen. In besonders bevorzugter Weise werden an elastischen Seitenteilen der Kniebandage unelastische Gurte angenäht, um ein Kräuseln des unelastischen Materials zu vermeiden.

Besonders bevorzugt ist das Verbindungselement so ausgestaltet, dass über das Verbindungselement zwei Schienen oder ein die Orthese verlängerndes Zusatzmodul mit einer Schiene befestigt werden kann. Die Befestigung des Zusatzmoduls oder der Schiene mit einem Verbindungselement ist dabei als kraft- und/oder formschlüssige Verbindung ausgestaltet. Entsprechend kann es sich bei der Verbindung zwischen dem Zusatzmodul bzw. der zusätzlichen Schiene mit dem Verbindungselement um eine Schraub-, Haken-Ösen-, Verrastung-, Ansteckverbindung, um einen Klettverschluss, wie beispielsweise Haken- und Flauschband (Filzband), Pilzkopfband und Veloursband Knöpfe, Pilzkopfband und Flauschband, Pilzkopfband auf Pilzkopfband oder extrudierte Haken/Pilze auf Wirkware oder um ein Ankleben etc. handeln. Als Zusatzmodul soll dabei ein Modul verstanden werden, welches die Orthese nach oben bis zur Hüfte eines Patienten und/oder bis zum Knöchelgelenk des Patienten verlängert.

Vorzugsweise ist das Verbindungselement symmetrisch ausgestaltet, so dass wahlweise ein oberes oder ein unteres Halteteil befestigt werden kann. Eine symmetrische Ausgestaltung des Verbindungselements ist zudem auch vorteilhaft, da dadurch der Zusammenbau der modularen Orthese erleichtert wird. Zudem eignet sich eine symmetrische Ausgestaltung des Verbindungselements auch dazu, Halteteile gleich- und/oder gegenläufig an der Schiene anzulegen, d. h. bspw. eine vordere und eine hintere Spange an dem symmetrisch ausgestalteten Verbindungselement zu befestigen.

In vorteilhafter Weise kann das Verbindungselement integral mit der Schiene aber auch integral mit dem Halteteil ausgestaltet sein. Natürlich ist auch eine Kombination von integral mit der Schiene oder integral mit dem Halteteil ausgestalteten Verbindungselementen denkbar.

Die vorliegende Offenbarung kann auch nur ein Gelenk für eine Orthese, insbesondere ein Orthesengelenk, umfassen und kann auch nur auf das Gelenk für die Orthese, insbesondere das Orthesengelenk, gerichtet werden mit den in der Beschreibung, der Figurenbeschreibung und den in den Figuren dargestellten vorteilhaften Ausführungen. Bevorzugt verbindet die als Komponente für die modular aufgebaute Orthese ausgestaltete Schiene zwei Halteteile gelenkig miteinander über ein Orthesengelenk. D. h. dass eine über das Verbindungselement mit der Schiene verbundene obere Spange über ein Orthesengelenk mit einer weiteren Schiene verbunden ist, an der über ein weiteres Verbindungselement ein weiteres Halteteil, nämlich bspw. eine Unterschenkelspange befestigt ist.

Zum Befestigen von bspw. Gurten kann die Schiene zusätzlich Befestigungselemente zum Befestigen von Aufnahmeelementen, hier insbesondere von Gurtaufnahmeelementen, aufweisen. Bei den Befestigungselementen zum Befestigen der Aufnahmeelemente handelt es sich vorzugsweise um kraft- und/oder formschlüssige Befestigungen, wie Klettverschlüsse, wie beispielsweise Haken- und Flauschband (Filzband), Pilzkopfband und Veloursband Knöpfe, Pilzkopfband und Flauschband, Pilzkopfband auf Pilzkopfband oder extrudierte Haken/Pilze auf Wirkware, Pilzköpfe, Schrauben, Verrastungen, Haken, Nieten, Klebverbindungen, Ausstanzen und Einhaken in ausgestanzte Löcher etc.. Wie auch bereits für das Aufnahmeelement an dem Verbindungselement beschrieben, kann es sich bei den Aufnahmeelementen für die Schiene auch um einen Schnellverschluss, umfassend wenigstens eine Schnalle und wenigstens eine Spange, handeln.

Um einen festen Sitz der Orthese über die Schienen an dem Patienten zu erlangen, und um anatomische, physiognomische und physiologische Besonderheiten des Patienten bzw. des Genesungszustands des Gelenks ausgleichen zu können, wobei insbesondere neben dem festen Sitz der Tragekomfort des Patienten im Vordergrund steht, können die Schienen konvex und/oder konkav ausgestaltete Bereiche aufweisen. So kann bspw. eine konvex ausgestaltete Schiene beispielhaft an eine O-Bein-Stellung eines Patienten angepasst sein. Bei dem Aufnahmeelement, welches an der Schiene oder dem Verbindungselement befestigt werden kann bzw. welches integral mit der Schiene oder dem Verbindungselement ausgestaltet sein kann, handelt es sich vorzugsweise um ein seitliches Gurtaufnahmeelement, an dem sich Gurte befestigen lassen, die neben den als Spangen ausgestalteten Halteteilen zum Anlegen der Orthese an einem Patienten dienen. In vorteilhafter Weise erfolgt die kraft- und/oder formschlüssige Verbindung, d. h. die Befestigung der Gurtaufnahmeelemente bspw. durch Steckelemente, mit/ohne Einrasten, sogenannte Steckpilze, Haken-Ösen-Verbindungen, Klettverschlüsse, wie beispielsweise Haken- und Flauschband (Filzband), Pilzkopfband und Veloursband Knöpfe, Pilzkopfband und Flauschband, Pilzkopfband auf Pilzkopfband oder extrudierte Haken/Pilze auf Wirkware, Druckknöpfe, Kleben etc.. Wie bereits für die anderen Komponenten der offenbarten modularen Orthese beschrieben, kann auch das Aufnahmeelement in unterschiedlichen Größen ausgestaltet sein, so dass bspw. für den kleineren Unterschenkel eines Patienten ein kleineres und für den meist breiteren Oberschenkel ein größeres Aufnahmeelement frei wählbar sein kann. Natürlich lässt sich auch ein kleineres Gurtaufnahmeelement bei der offenbarten modularen Orthese bei Patienten mit sehr schmalen Oberschenkeln einsetzen.

Wie bereits beschrieben kann das Aufnahmeelement mit einem ein- und/oder mehrteiligen Schnellverschluss verbindbar sein, um bspw. ein schnelles Öffnen oder Schließen der Orthese zu erleichtern. Darüber hinaus kann das Aufnahmeelement aber auch integral mit einem ein- und/oder mehrteiligen Schnellverschluss ausgestaltet sein. Das bedeutet, dass das Aufnahmeelement einen Schnellverschluss bildet. Der Schnellverschluss umfasst dabei wenigstens eine Schlaufe, die mit einer einstellbaren Schnalle verbunden ist, wobei bspw. ein Gurt an der Schlaufe befestigt ist, und so durch Schließen der Schnalle der Gurt an den Patienten anlegbar ist. Durch eine Rasterschiene an der Schlaufe, z. B. nach dem Schnellverschlussprinzip ähnlich dem eines Skischuhschnellverschlusses, ist es bei den genannten Schnellverschlüssen möglich, eine Justierung, d. h. eine Nachjustierung bspw. eines Gurtes problemlos vorzunehmen.

In Bezug auf die Befestigung des Aufnahmeelementes, bspw. einer Schraubverbindung, kann das Aufnahmeelement symmetrisch oder asymmetrisch ausgestaltet sein. In bevorzugter Weise bietet es sich an, das Aufnahmeelement asymmetrisch auszugestalten, damit Aufnahmeelemente zur Aufnahme eines Gurtes, die an zwei beabstandeten Befestigungselementen an der Schiene oder dem Verbindungselement befestigt sind, so an der Schiene oder dem Befestigungselement angelegt sind, dass der damit befestigte Gurt verwindungsfrei, d. h. parallel laufend befestigt werden kann.

In noch bevorzugter Weise umgreift das Aufnahmeelement zumindest abschnittsweise das Verbindungselement bzw. die Schiene und ist in vorteilhafter Weise kraft- und/oder formschlüssig mit dem Verbindungselement und/oder der Schiene verbunden. Ein in dieser Art ausgestaltetes Aufnahmeelement kann so in vorteilhafter Weise an dem Patienten zur Anlage kommen und so ein auf den Patienten durch die Schiene oder das Verbindungselement ausgeübter Druck durch die Anlage des Aufnahmeelementes an dem Patienten verhindert bzw. minimiert werden. In vorteilhafter Weise weist dazu das Aufnahmeelement Flügel auf, die die Fläche des Aufnahmeelements, die an dem Patienten anliegt, vergrößert. Somit kann der an dem Patienten anliegende Druck über die durch die Flügel vergrößerte Fläche des Aufnahmeelements verteilt werden. In vorteilhafter Weise ist dabei die Befestigung für ein weiteres Halteteil, nämlich hier in Form eines Gurtes, an dem dem Verbindungselement und/oder der Schiene abgewandten Ende des Aufnahmeelements vorgesehen. Zudem eignen sich die Flügel in unterschiedlichen Größen, die seitlich an dem Aufnahmeelement individuell austauschbar befestigt werden können, bzw. die integral mit dem Aufnahmeelement ausgestaltet sind, dazu, um das Aufnahmeelement an die individuelle Anatomie eines Patienten anzupassen.

Zur Befestigung des Aufnahmeelements und zur Anbindung des in Form eines Flügels oder beidseitig des Aufnahmeelements ausgebildeten Flügeln ausgestalteten Anlageelements weist das Verbindungselement und/oder die Schiene in vorteilhafter Weise eine Führung auf, an der das Aufnahmeelement führbar ist. Dadurch wird ein Verrutschen des Aufnahmeelements verhindert und dadurch dessen Funktion, nämlich die Druckminimierung der Schiene oder des Verbindungselements auf den Patienten bestmöglich unterstützt.

Zudem können die Führungen vorteilhafter Weise Verrastungen aufweisen, über die das Aufnahmeelement kraft- und/oder formschlüssig mit der Führung verbindbar ist und somit das Aufnahmeelement mit der Schiene oder dem Verbindungselement befestigt werden kann. Aber auch die Schiene oder auch das Verbindungselement können selber eine wellenförmige oder Raststruktur aufweisen, über die das Aufnahmeelement schiebbar ist und darüber kraft- und/oder formschlüssig zur Anlage gelangt.

Ist das Aufnahmeelement wie vorliegend beschrieben über das Verbindungselement bzw. die Schiene geführt, weist dieses zum Durchführen und/oder zum Einsetzen eines Halteteils in das Verbindungselement vorteilhafter Weise eine Ausnehmung auf, die es erlaubt, bspw. eine Spange durch die Ausnehmung des Aufnahmeelements in die Ausnehmung des Verbindungselements zu führen.

Um die Auflagefläche der Anlageelemente, hier vorzugsweise der Flügel des Aufnahmeelements zu vergrößern, sind diese in vorteilhafter Weise nach unten oder nach oben hin entlang der Schiene vergrößert. So kann bspw. ein Anlageelement als zwei übereinander liegende und beidseitig des Verbindungselements und/oder der Schiene ausgestalteten Flächen bestehen, insbesondere in Form von Flügeln. Natürlich können die Flügel, d. h. die Anlageelemente einzeln an dem Aufnahmeelement befestigt sein, beispielsweise an einem von dem Aufnahmeelement gebildeten Mittelsteg.

Insbesondere für die Verwendung der modularen Orthese als OA-Orthese weist das Aufnahmeelement ein flexibles Mittelteil auf, oder bspw. eine gelenkige Verbindung, um insbesondere in Verbindung mit dem gelenkig ausgestalteten Verbindungselement und der als Scharnier ausgeführten Schiene eine Schieneneinstellung der OA-Orthese zu erlauben. Um Verschmutzungen der Orthese im Bereich der Befestigungselemente, den Schnellverschlüssen und bspw. der Ausnehmungen und Aufnahmen zu verhindern, sind diese vorzugsweise durch zumindest eine Abdeckung abgedeckt. Zudem dient die Abdeckung auch dazu, die Orthese zu individualisieren, d. h. zur Nutzung als Werbefläche. Zur gelenkigen Verbindung der Schienen, die über das Verbindungselement mit den Halteteilen verbunden sind, dient in bevorzugter Weise ein Gelenk, insbesondere ein Orthesengelenk, welches zwei mit den Schienen oder den Schenkelteilen verbundene Schenkelenden, die aufeinander zugerichtet sind, gelenkig, d. h. schwenkbar miteinander verbindet. Dabei weist eines der Schenkelenden bevorzugt eine Lagerausnehmung auf, die vorzugsweise als Führungskulisse ausgebildet ist, in die ein Gelenkeinsatz eingreift und dieser mit dem anderen Schenkelende, welches keine Lagerausnehmung aufweist, durch Durchführung von Befestigungselementen durch den Gelenkeinsatz und durch die Lagerausnehmung befestigt ist. Dabei sind der Gelenkeinsatz und die als Führungskulisse ausgestaltete Lagerausnehmung so ausgebildet, dass die Schienen in unterschiedlichen Drehstellungen, d. h. relativ zueinander unterschiedliche Winkel einschließend, feststellbar sind. Der Gelenkeinsatz ist bevorzugt als Kreisscheibe ausgestaltet mit einer Umfangsverzahnung, die der Innenverzahnung der Lagerausnehmung des Schenkelendes der einen Schiene entspricht.

Um eine Einstellung des Gelenks über einen bestimmten Winkel hinaus zu verhindern, sind an den Schenkelenden oder dem Gelenkeinsatz vorteilhaft begrenzende Anschläge ausgestaltet. Besonders bevorzugt sind begrenzende Anschläge durch die für den Gelenkeinsatz vorgesehene Lagerausnehmung, die als Führungskulissen den gegenseitigen Drehwinkel der Schienen begrenzen, gebildet.

Zur Einstellung des Gelenks für die offenbarten Orthese sind bevorzugt an der Umfangsverzahnung oder zumindest im Bereich der Umfangsverzahnung und/oder der Innenverzahnung bzw. auf dem Gelenkeinsatz zur Einstellung des Drehwinkels der Schienen Markierungen vorgesehen.

Zur Erleichterung des Zusammenbaus und eventuell zum Wechsel des Gelenks oder des Gelenkeinsatzes ist der Gelenkeinsatz bevorzugt aus zwei ineinander greifenden Teilen zusammengesetzt. Die so ineinander greifenden Teile des Gelenkeinsatzes bilden dabei die Kreisscheibe mit Umfangsverzahnung. Besonders bevorzugt sind die Teile des Gelenkeinsatzes in so genannter Ying-Yang-Form ausgestaltet, wobei die beiden Teile kraft- und/oder formschlüssig ineinander greifen. Besonders bevorzugt weisen die beiden Teile jeweils einen Zapfen und eine Aussparung auf, wobei die Aussparung zur form- und/oder kraftschlüssigen Aufnahme des Zapfens des anderen Teils und der Zapfen zum Einsetzen in die Aussparung des anderen Teils dient.

Um das Gelenk für die Orthese mit dem Verbindungselement mit einer Zusatzfunktion zu belegen, kann diese mit einer Sit-/Go-Sperre in Form eines Druckstellers ausgestaltet sein. Dabei weist vorteilhaft wenigstens ein Deckelelement, welches zumindest abschnittsweise das Gelenk abdeckt, einen darauf angeordneten Drucksteller auf, der mit einem Sperrelement zur Arretierung des Gelenks in Wirkverbindung steht. Dabei kann der Drucksteller bereits mit dem Gelenk ausgestaltet sein oder dieser kann auch modular als Sit-/Go-Sperre, beispielsweise zu einem späteren Zeitpunkt einsetzbar sein. Die Sit-/Go-Sperre ermöglicht eine Immobilisierungsfunktion (Geh-Funktion) die in vorteilhafter Weise die normalerweise bisher übliche Immobilisationsschiene ersetzt, wobei die Sit-Funktion, d. h. über die Entsperrung des Gelenks ein Sitzen, d. h. ein Beugen des Gelenks ermöglicht. Die Immobilisationssperre, d. h. die Go-Sperre, für die Geh-Funktion soll dabei nicht nur als Immobilisierungsfunktion für die Geh-Funktion verstanden werden, sondern soll auch für die unterschiedlichsten Bewegungseinschränkungen des Orthesengelenks dienen. Als Arretierung für die Sit-/Go-Sperre, d. h. bei Verwendung eines Druckstellers kann bspw. ein Stift dienen, der durch Druck auf den Drucksteller in eine Innenkulisse des Gelenkeinsatzes greift, wodurch eine Drehung des Gelenkeinsatzes, d. h. eine Drehung des Gelenks unterbunden wird.

Die vorliegende Offenbarung umfasst auch nur ein Gelenkpolster für eine Orthese und kann auch nur auf das Gelenkpolster für die Orthese gerichtet werden mit den in der Beschreibung, der Figurenbeschreibung und den in den Figuren dargestellten vorteilhaften Ausführungen.

In vorteilhafter Weise ist eine weitere Anpassung, d. h. eine individuelle und spezielle Anpassung der Orthese durch ein an dem Gelenk ausgestaltetes Gelenkpolster, ein sogenanntes Kondylenpolster möglich, welches sich der Physiognomie und/oder der Pathologie eines durch die Orthese stabilisierten Gelenks anpassen lässt.

Eine Anpassung der Gelenkpolster an die Physiognomie bzw. Pathologie des durch die Orthese gestützten Gelenks erfolgt in bevorzugter Weise durch kaskadierbare Gelenkpolster, d. h. durch an-, in-, oder aufeinander steckbare Polster, die bspw. zudem unterschiedliche Stärkegrade aufweisen können.

Um die Anpassung des Gelenkpolsters an die Physiognomie und/oder Pathologie des Knies noch zu verbessern bzw. noch zu erleichtern, ist das Polster in besonders bevorzugter Weise fluidtechnisch beaufschlagbar. Die fluidtechnische Beaufschlagung schließt das Befüllen des Polsters mit Gas, Luft, Flüssigkeiten oder Gas-Luft-Flüssigkeitsgemischen ein. Je nach Bedarf kann dadurch bei einem fluidtechnisch beaufschlagten Polster das Gas, die Luft, die Flüssigkeit oder das Gas-Luft-Flüssigkeitsgemisch aufgeschlagen bzw. abgeführt werden, ohne dabei das Gelenkpolster aus seiner Anlage an dem durch die Orthese stabilisierten Gelenk zu befreien.

Sowohl die modular aufgebaute Orthese, das Halteteil, die Schiene, das Aufnahmeelement, das Gelenk und die Gelenkpolster werden einzeln für sich oder in beliebiger Kombination der von zwei oder mehr Komponenten als zusätzliche Aspekte zu dem Verbindungselement betrachtet.

Um Wiederholungen bzgl. weiterer Vorteile der Komponenten für die offenbarte modular aufgebaute Orthese zu vermeiden wird auf die Beschreibung der vorteilhaften Ausgestaltung der modular aufgebauten Orthese bzw. jeweils auf die Beschreibung der vorteilhaften Ausgestaltungen der einzelnen Komponenten für die modular aufgebaute Orthese verwiesen und es wird vollumfänglich und jeweils umgekehrt geltend auf diese zurückgegriffen.

Weitere, die Offenbarung verbessernde Maßnahmen werden nachstehend mit der Beschreibung von bevorzugten Ausführungsbeispielen der Offenbarung anhand der Figuren näher dargestellt. Dabei können die in den Ansprüchen und in der Beschreibung erwähnten Merkmale jeweils einzeln für sich oder in beliebiger Kombination offenbarungswesentlich sein. Dabei ist zu beachten, dass die Figuren nur beschreibenden Charakter haben und nicht dazu gedacht sind, die Offenbarung in irgendeiner Form einzuschränken. Es zeigen:
- Figur 1: eine modular aufgebaute Orthese in einer perspektivischen Seitenansicht,
- Figur 2: die modular aufgebaute Orthese aus Figur 1 in einer Explosionsansicht,
- Figur 3: die Orthese aus Figur 1 in einer Frontalansicht,
- Figur 4: das in Figur 2 gezeigte Orthesengelenk in einer Detailansicht,
- Figur 5: ein als Anlageelement ausgestaltetes Aufnahmeelement als Komponente für die modular aufgebaute Orthese,
- Figur 6: ein mit einer Sit-/Go-Sperre ausgestaltetes Orthesengelenk,
- Figur 7a+7b: in einer seitlichen Schnittansicht die Sit-Funktion und die Go-Funktion der Sit-/Go-Sperre aus Figur 6,
- Figur 8: eine Ausführungsform eines gelenkigen Verbindungselement mit verstellbarem Scharniergelenk,
- Figur 9: das Verbindungselement aus Figur 8 mit einem seitlichen Stellknopf,
- Figur 10: das Verbindungselement aus Figur 8, welches mit einem Werkzeug einstellbar ist,
- Figur 11: das Verbindungselement aus Figur 9, wobei der seitliche Stellknopf durch eine mittels Werkzeug bedienbaren Schraubenkopf ersetzt ist,
- Figur 12: das Verbindungselement aus Figur 9 und 11, wobei der seitliche Stellknopf oder der Schraubenkopf durch eine Kurbel ersetzt ist,
- Figur 13: das Verbindungselement aus Figur 8 mit über eine Rändelwelle verstellbarem Scharniergelenk,
- Figur 14: eine weitere Ausführungsform eines Verbindungselementes mit flexiblem Mittelteil,
- Figur 15: das Verbindungselement aus Figur 14 mit einem auf das flexible Mittelteil aufgesetzten Fixier-Clip,
- Figur 16: das Verbindungselement aus Figur 14 mit seitlich an das flexible Mittelteil aufgesetzten Fixier-Clips,
- Figur 17: das Verbindungselement aus Figur 14 mit einer auf das flexible Mittelteil aufgesetzten Winkelspange,
- Figur 18: das Verbindungselement aus Figur 14 mit einem seitlich auf-/eingesetzten Stabilisator,
- Figur 19: zwei Verbindungselemente mit Scharniergelenk, die jeweils mit einem Schienenteil einer Gelenkschiene verbunden sind, wobei das Gelenk der Gelenkschiene zwei Scharniergelenke aufweist,
- Figur 20: zwei Verbindungselement mit Scharniergelenk, die jeweils mit einem Schienenteil einer Gelenkschiene verbunden sind, wobei die Schienenteile jeweils ein Scharniergelenk aufweist,
- Figur 21: eine Gelenkschiene, die über Verbindungselemente mit den Halteteilen der Orthese verbunden ist, wobei die Schienenteile zwischen dem Gelenk und dem Verbindungselement jeweils zwei Scharniergelenke aufweisen,
- Figur 22: die Gelenkschiene aus Figur 21 mit nur einem Scharniergelenk,
- Figur 23: eine alternative Ausführungsform eines Verbindungselementes, das zum Einlegen der Schiene in die Aufnahme geöffnet werden kann,
- Figur 24: das Verbindungselement aus Figur 23 mit einem an das in der Aufnahme eingelegten Schienenteil angeklipsten Gelenk und
- Figur 25: das Verbindungselement aus Figur 24 mit einem zwischen dem Orthesengelenk und dem Schienenteil eingesetzten Zwischenelement mit Scharniergelenk.

In den unterschiedlichen Figuren sind gleiche Teile stets mit denselben Bezugszeichen versehen, weshalb diese in der Regel nur einmal beschrieben werden.

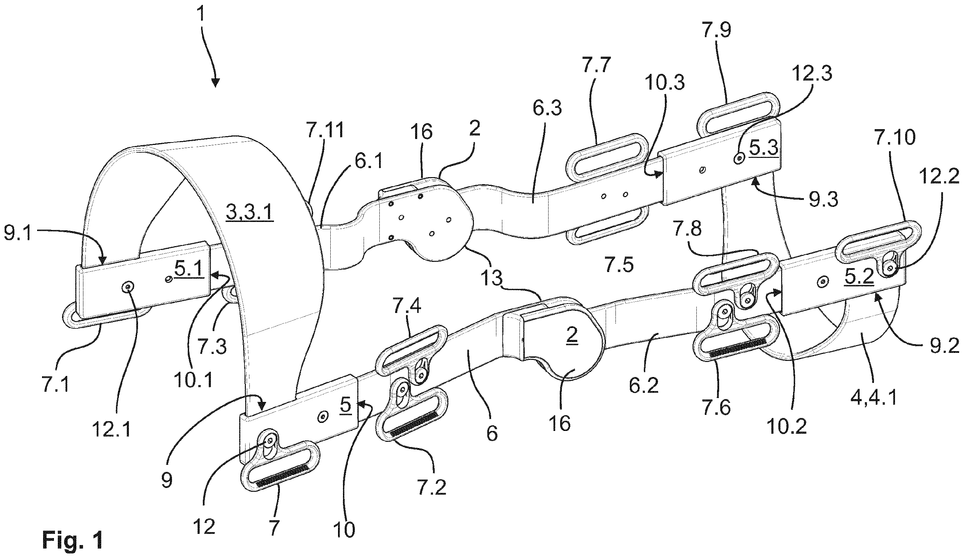

Figur 1 zeigt in perspektivischer Seitenansicht eine modular aufgebaute Orthese 1, die vorliegend als Knieorthese mit parallel zueinander liegenden Schienen 6, 6.1, 6.2, 6.3 ausgestaltet ist, wobei die Schienen bzw. die Schienenschenkel 6 und 6.2 und die Schienen bzw. Schienenschenkel 6.1 und 6.3 über jeweils ein Orthesengelenk 2 miteinander verbunden sind. Des Weiteren weist die Orthese 1 ein erstes Halteteil 3 in Form einer Spange 3.1 auf, welche zur Anlage an einem Oberschenkel eines Patienten gelangt. Ein weiteres zweites Halteteil 4, welches ebenfalls als Spange 4.1 ausgestaltet ist, dient zur Anlage der Orthese 1 an den Unterschenkel eines Patienten. Vorliegend sind die Spangen 3.1 und 4.1 gegenläufig, d. h. als vordere, den Oberschenkel teilweise umfassende Spange 3.1, und als hintere, den Unterschenkel des Patienten zumindest abschnittsweise umgreifende Spange 4.1, ausgestaltet. Als eine weitere Komponente umfasst die Orthese 1 Verbindungselemente 5, 5.1, 5.2, 5.3 über die die Halteteile 3 und 4 an den Schienen 6, 6.1, 6.2, 6.3 befestigt sind. Im Konkreten dient das Verbindungselement 5 dazu, die Schiene 6 in der Aufnahme 10 aufzunehmen. Über die Ausnehmung 9 ist ein Ende des Halteteils 3, nämlich hier der Oberschenkelspange 3.1 in das Verbindungselement 5 geführt und über die Befestigung 12 mit der Schiene und dem Verbindungselement befestigt. Die parallel zur Schiene 6 liegende Schiene 6.1 ist über die Aufnahme 10.1 des Verbindungselements 5.1 in das Verbindungselement 5.1 geschoben. Das andere Ende des Halteteils 3, nämlich der Spange 3.1, welches nicht in das Verbindungselement 5 eingeführt ist, ist durch die Ausnehmung 9.1 in das Verbindungselement 5.1 eingeführt und ist über das Befestigungselement 12.1 mit der Schiene und dem Verbindungselement verbunden. Somit verbindet das Halteteil 3 nämlich die Oberschenkelspange 3.1 die Schienen 6 und 6.1 miteinander.

Die Schiene 6.2 ist über die Aufnahme 10.2 mit dem Verbindungselement 5.2 verbunden. In die Ausnehmung 9.2 des Verbindungselements 5.2 ist ein Ende des Halteteils 4, welches hier als Unterschenkelspange 4.1 ausgestaltet ist, eingeführt und über das Befestigungselement 12.2 mit dem Verbindungselement 5.2 und der Schiene 6.2 verbunden. Das andere Ende des Halteteils 4 ist über die Ausnehmung 9.3 des Verbindungselements 5.3 in das Verbindungselement 5.3 eingeführt und mit dem Befestigungselement 12.3 mit dem Verbindungselement 5.3 und der Schiene 6.3, die über die Aufnahme 10.3 mit dem Verbindungselement 5.3 verbunden ist, befestigt. Sowohl das als Oberschenkelspange 3.1 ausgestaltete Halteteil 3 als auch das als Unterschenkelspange 4.1 ausgestaltete Halteteil 4 kann durch Lösen der Befestigungselemente 12, 12.1, 12.2 und 12.3 gelöst und ggf. durch eine andere Spange 3.1 und 4.1, die der Größe, d. h. dem Umfang des Ober- bzw. des Unterschenkels eines Patienten angepasst ist, ausgetauscht werden. Natürlich ist es auch denkbar, dass bei Patienten mit einem geringen Oberschenkelumfang zwei Unterschenkelspangen 4.1 eingesetzt werden, nämlich das Halteteil 3, welches hier als Oberschenkelspange 3.1 ausgestaltet ist, durch eine Unterschenkelspange 4.1 ersetzt werden. Über die Befestigungselemente 12, 12.1, 12.2 und 12.3 sind zudem Aufnahmeelemente 7, 7.1, 7.9 und 7.10 an dem Verbindungselement befestigt. Die Aufnahmeelemente 7, 7.1, 7.9, 7.10 sowie auch die weiteren Aufnahmeelemente 7.2, 7.3, 7.4, 7.5, 7.6, 7.7, 7.8 und 7.11 sind als Gurtaufnahmeelemente ausgestaltet, die zur Befestigung von Gurten dienen, die die Anlage der Orthese 1 an einen Patienten neben den Halteteilen 3 und 4, die vorliegend als Spangen 3.1 und 4.1 ausgestaltet sind, zusätzlich stabilisieren.

Wie beispielhaft an den Aufnahmeelementen 7.2 und 7.4 dargestellt, sind diese bezogen auf ihre Befestigung asymmetrisch ausgestaltet, d. h. dass diese an zwei nebeneinander liegenden Befestigungen so zueinander ausgerichtet werden können, dass die Aufnahmebereiche zur Aufnahme des Gurtes auf einer Linie zueinander stehen.

Wie anhand der Schiene 6.1 zu erkennen ist, weist diese vor dem Orthesengelenk 2 einen konkaven und einen konvexen Abschnitt auf. Die Schiene 6.3 schließt an das Orthesengelenk mit einem konvexen Abschnitt an, der in einen konkaven Abschnitt der Schiene 6.3 übergeht. Diese Ausgestaltung der Schienen 6.1 und 6.3 dient dazu, die Orthese 1 passgenau an das Kniegelenk eines Patienten anzupassen. Natürlich können auch die Schienen 6 und 6.2 konkave und konvexe Bereiche aufweisen.

Insgesamt sind die in der Figur 1 dargestellten Komponenten der modular aufgebauten Orthese 1 durch an die Größe und die spezielle Indikation der Orthese 1 angepasste Komponenten austauschbar.

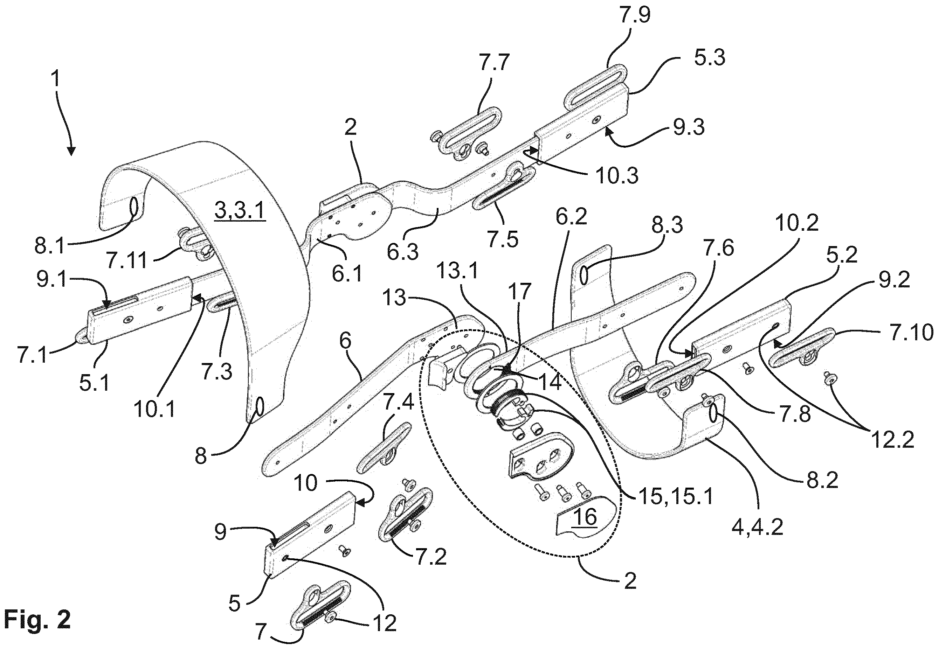

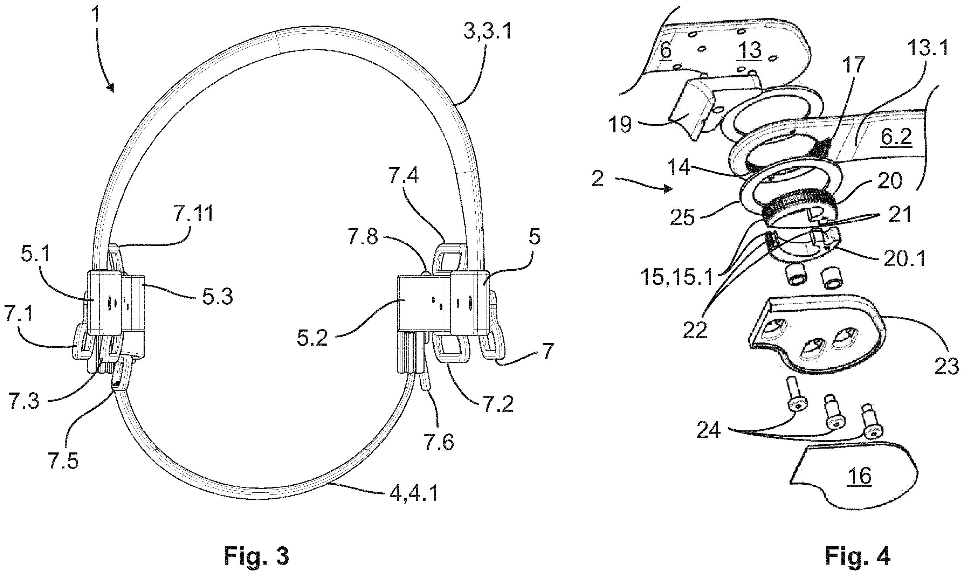

Figur 2 zeigt die Orthese 1 aus Figur 1 in einer Explosionszeichnung. Wie zu erkennen ist, weisen die als Halteteile 3 und 4 ausgestalteten Spangen 3.1 und 4.2 an ihren Enden die durch die Ausnehmungen 9, 9.1, 9.2 und 9.3 in die Verbindungselemente 5, 5.1, 5.2 und 5.3 geführt werden, Ausnehmungen 8, 8.1, 8.2, und 8.3 in Form von Langlöchern auf. Die als Langlöcher ausgestalteten Ausnehmungen 8, 8.1, 8.2 und 8.3 erlauben ein Justieren und eine geringfügige Weitenverstellung der als Spangen 3.1 und 4.1 ausgestalteten Halteteile 3 und 4 in den Verbindungselementen 5, 5.1, 5.2 und 5.3. Nach Einführen der Enden der Spangen 3.1 und 4.1 in die Ausnehmungen 9, 9.1, 9.2 und 9.3 der Verbindungselemente 5, 5.1, 5.2 und 5.3 und nach Einführen der Schienen 6, 6.1, 6.2 und 6.3 über die Aufnahmen 10, 10.1, 10.2 und 10.3 der Verbindungselemente, wobei die genannten Schritte auch in einer anderen Reihenfolge erfolgen können, werden die Befestigungselemente 12, 12.1, 12.2 und 12.3 in die Verbindungselemente 5, 5.1, 5.2 und 5.3 eingeführt und dabei durch die Ausnehmungen 8, 8.1, 8.2 und 8.3 geführt. Entsprechend können die Halteteile 3 und 4 hier vorliegend die Spangen 3.1 und 4.1 relativ zu den Befestigungselementen 12, 12.1, 12.2 und 12.3 entlang der Ausnehmungen 8, 8.1, 8.2 und 8.3 relativ zu den Befestigungselementen 12, 12.1, 12.2 und 12.3 bewegt werden. Das Gelenk 2, welches im Detail in Figur 4 dargestellt ist, wird hier beispielhaft anhand des in der Figur vorne liegenden Gelenks 2 beschrieben, welches die Schienen 6 und 6.2 schwenkbar miteinander verbindet. Mit den Schienen 6 und 6.2 sind zwei aufeinander zugerichtete Schenkelenden 13 und 13.1 verbunden. Das Schenkelende 13.1 weist eine Lagerausnehmung 14 in Form einer Führungskulisse auf. Die Lagerausnehmung 14 weist dabei eine Innenverzahnung auf, an dessen Umfangsbereich auf dem Schenkelende 13.1 eine Markierung 17 ausgestaltet ist, die zur Einstellung des Drehwinkels der Schienen 6 und 6.1 dient. In die Innenverzahnung der Lagerausnehmung 14 greift ein zweiteilig ausgestalteter Gelenkeinsatz 15, der als eine aus zwei ineinander greifenden Teilen ausgestaltete Kreisscheibe 15.1 ausgestaltet ist. Die hier in der Figur 2 beschriebenen Merkmale des Gelenks 2 dienen zur Orientierung des Gelenks 2 in der modular aufgebauten Orthese 1. Weitere Einzelheiten und Merkmale des Gelenks 2 werden nachfolgend anhand der Figur 4 genauer erläutert.

Figur 3 zeigt die modular aufgebaute Orthese 1 aus Figur 1 in einer Frontalansicht, nämlich von vorne auf das als Oberschenkelspange 3.1 ausgestaltete Halteteil 3. Wie zu erkennen ist, sind das Halteteil 3 und das Halteteil 4 nämlich hier die Oberschenkelspange 3.1 und die Unterschenkelspange 4.1 gegenläufig zueinander, nämlich als vordere Spange 3.1 und als hintere Spange 4.1 befestigt. Durch die konvex bzw. konkav ausgestalteten Bereiche der Schienen 6, 6.1, 6.2 und 6.3 wird zudem eine Verlagerung des oberen Halteteils 3 relativ zu dem unteren Halteteil 4, vorliegend nach rechts in der Darstellung 3 erreicht. Entsprechend kann die hier dargestellte Orthese 1 beispielhaft für einen Patienten mit O-Beinstellung eingesetzt werden. Zudem stellt Figur 3 eindrucksvoll dar, wie dem Umfang eines Oberschenkels relativ zu dem Umfang eines Unterschenkels eines Patienten durch unterschiedlich große Spangen 3.1 und 4.1 Rechnung getragen wird.

Figur 4 zeigt ein Gelenk für eine Orthese 2, welches über zwei Schienen 6 und 6.2 ein erstes und ein zweites Halteteil 3 und 4 verbindet. Das Gelenk umfasst zwei aufeinander zugerichtete Schenkelenden 13 und 13.1, die Bestandteil der Schienen 6 und 6.2 sind. Das Schenkelende 13.1 weist eine Lagerausnehmung 14 mit einer Innenverzahnung auf, die als Führungskulisse für den Gelenkeinsatz 15 dient, der hier vorliegend als Kreisscheibe 15.1 dargestellt ist, die aus zwei Teilen 20 und 20.1 besteht, die ähnlich dem Ying-Yang-Prinzip ineinander greifen. Dazu weist das Teil 20 Zapfen 21 auf, die in Aussparungen 22 des anderen Teils 20.1 form- und kraftschlüssig fassen. Natürlich können die Aussparungen 22 und die Zapfen 21 auch so an den Teilen 20 und 20.1 ausgestaltet sein, dass jeweils ein Teil 20 oder 20.1 einen Zapfen 21 und eine Aussparung 22 aufweist, in die die Aussparung 22 bzw. der Zapfen 21 des anderen Teils formschlüssig greift. Um eine genaue Einstellung des Drehwinkels der Orthese 1 über das Gelenk 2 zu ermöglichen, sind am Außenrand der Innenverzahnung der Lagerausnehmung 14 an dem Schenkelende 13.1 der Schiene 6.2 Markierungen vorgesehen, die die Winkelstellung der Schienen 6 und 6.2 über das Gelenk 2 anzeigt. Zur Begrenzung des Drehwinkels des Gelenks 2 ist an dem Schenkelende 13 der Schiene 6 ein Anschlag 19 angeordnet. Der Anschlag 19 wirkt dabei so mit dem weiteren Bauteil 23 des Gelenks 2 zusammen, dass dieses abschnittsweise entlang dessen Außenkontur mit dem daran befestigen Gelenkeinsatz 15 bei Drehung des Gelenkeinsatzes in der als Führungskulisse ausgestalteten Ausnehmung am Schenkelende 13.1 der Schiene 5.2 drehbar ist, und ab einem gewissen Drehwinkel so mit seiner Außenkontur zur Anlage an den Anschlag 19 gelangt, dass eine weitere Drehung des Gelenks 2 nicht mehr möglich ist. Wie dargestellt, dienen drei Befestigungselemente 24, die durch das Bauteil 23, durch den Gelenkeinsatz 15 und die Lagerausnehmung 14 geführt werden, zur Befestigung der Komponenten des Gelenks 2, d. h. zu dessen Befestigung an dem Schenkelende 13 der Schiene 6. Zudem dienen zwei Lagerscheiben 25, die zum einen zwischen den Schenkelenden 13 und 13.1 und zum anderen zwischen dem Schenkelende 13.1 und dem weiteren Bauteil 23 angeordnet sind, dazu, dass die durch die Lagerscheiben 25 voneinander getrennten Teile relativ zueinander drehbar sind. Über ein Deckelelement 16 werden sowohl die Befestigungselemente 24 als auch der Gelenkeinsatz 15 und die Lagerausnehmung 14 abgedeckt und so vor Schmutz bewahrt. Neben der Funktion eines begrenzenden Anschlags dient der Anschlag 19 auch dazu, die Schenkelenden 13 und 13.1 so voneinander zu beabstanden, dass genügend Bauraum geschaffen wird, um die Lagerschalen 25, das Schenkelende 13.1 mit der Lagerausnehmung 14 und den Gelenkeinsatz 15 zwischen dem Schenkelende 13 und dem Bauteil 23 des Gelenks 2 aufzunehmen.

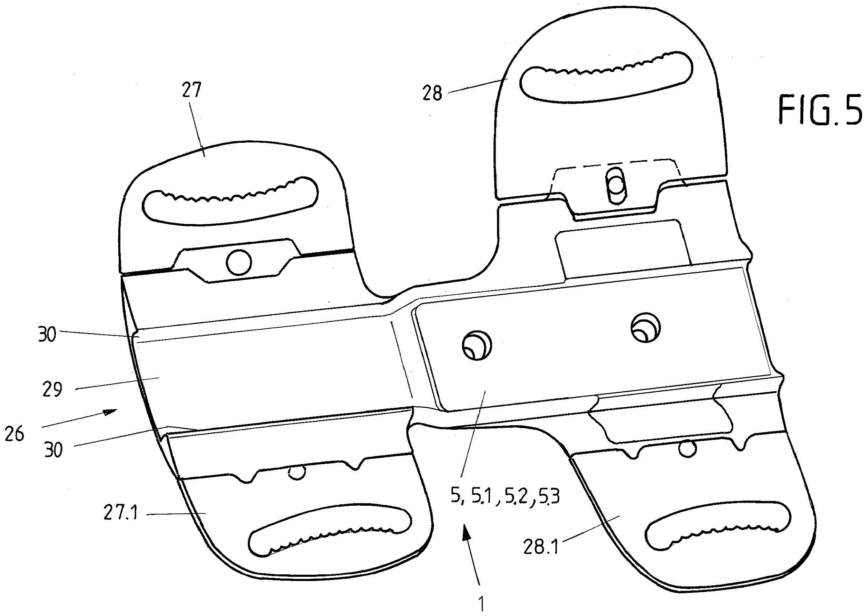

Figur 5 zeigt ein als Anlageelement 26 ausgestaltetes Aufnahmeelement 7, 7.1, 7.2, 7.3, 7.4, 7.5, 7.6, 7.7, 7.8, 7.9, 7.10, 7.11, welches zur zusätzlichen Befestigung von Halteteilen 3 und 4, hier insbesondere in Form von seitlichen Flügeln 27, 27.1 und 28, 28.1 ausgestaltet ist. Die Flügel 27, 27.1 und 28, 28.1 sind als Schnallen ausgestaltet, die zur Aufnahme von Gurten dienen. Dabei bilden die Flügel 27 und 27.1 und die Flügel 28 und 28.1 jeweils ein Flügelpaar, welches zur Befestigung eines Halteteils 3 und 4, insbesondere zur Befestigung wenigstens eines Gurtes dient. Insgesamt besteht das Anlageelement 26 aus einem ein Verbindungselement 5, 5.1, 5.2 und 5.3 zumindest abschnittsweise umgreifenden Mittelsteg 29, der beidseitig seitliche Führungen 30 aufweist, an denen das Verbindungselement 5, 5.1, 5.2 und 5.3 sowie eine in dem Verbindungselement 5, 5.1, 5.2 und 5.3 aufgenommene Schiene 6, 6.1, 6.2 und 6.3, die zur besseren Übersicht in Figur 5 nicht dargestellt ist, führbar sind. Dabei können die Führungen 30, des Verbindungselements 5, 5.1, 5.2 und 5.3 und/oder die Schiene 6, 6.1, 6.2 und 6.3 Verrastungen aufweisen, an denen das Anlageelement 26 kraft-und/oder formschlüssig über mit dem Verbindungselement 5, 5.1., 5.2 und 5.3 oder der Schiene 6, 6.1, 6.2 und 6.3 befestigbar ist. Natürlich kann eine formschlüssige Verbindung bzw. Befestigung zwischen dem Verbindungselement 5, 5.1, 5.2 und 5.3 bzw. der Schiene 6, 6.1, 6.2 und 6.3 und dem als Anlageelement 26 ausgestalteten Aufnahmeelement 7, 7.1, 7.2, 7.3, 7.4, 7.5, 7.6, 7.7, 7.8, 7.9, 7.10, 7.11 auch durch eine andere kraft- und/oder formschlüssige Verbindung erfolgen, d. h. die Befestigung des Anlageelements bspw. durch Steckelemente, mit und ohne Einrasten, so genannte Steckpilze, Haken-Ösen-Verbindungen, Klettverschlüsse, wie beispielsweise Haken- und Flauschband (Filzband), Pilzkopfband und Veloursband Knöpfe, Pilzkopfband und Flauschband, Pilzkopfband auf Pilzkopfband oder extrudierte Haken/Pilze auf Wirkware, Druckknöpfe, Kleben, Schrauben, Vernieten etc. erfolgen. Wie in der Figur dargestellt, erfolgt seitlich der Führungen 30 die Befestigung der als Schnallen, d. h. als Gurtaufnahmeelemente ausgestalteten Flügel 27, 27.1 und 28, 28.1 über Steckelemente, d. h. dass die Flügel 27, 27.1 und 28, 28.1 lösbar an dem Mittelsteg 29 der Führung 30 befestigt sind. Entsprechend können bspw. dem Radius eines Oberschenkel bzw. eines Unterschenkels eines Patienten angepasst die Flügel 27, 27.1 und 28, 28.1 bezüglich ihrer Größe und beispielsweise bezüglich ihres Radius ausgewählt werden. Der als Gurtaufnahmeelement ausgestaltete Flügel 27, 27.1 lässt sich bspw. zur Befestigung eines Gurtes, welcher als zusätzliches Halteteil für die modular aufgebaute Orthese 1 dient, für einen etwas schmaleren Oberschenkel oder sich verjüngenden Teil eines Oberschenkels bzw. eines Unterschenkels eines Patienten einsetzen. Dagegen kann der als Gurtaufnahmeelement ausgestaltete Flügel 28, 28.1 beispielhaft zur Aufnahme eines Gurtes im Bereich des Oberschenkels eines Patienten dienen, da der Flügel 28, 28.1 einen größeren Radius als der Flügel 27, 27.1 aufweist, und so einem größeren Radius, beispielsweise dem Oberschenkelumfang eines Patienten Rechnung tragend für die Aufnahme eines Gurtes an der modular aufgebauten Orthese 1 zur Anlage an einem Oberschenkel, verwendet werden. Natürlich ist es auch denkbar, die Flügel 27, 27.1 und 28, 28.1 oder anders ausgestaltete Flügel, die bspw. auch als zusätzliche Aufnahmeelemente 7, 7.1, 7.2, 7.3, 7.4, 7.5, 7.6, 7.7, 7.8, 7.9, 7.10, 7.11 für weitere Halteteile 3 und 4, die bspw. als Spange 3.1 und 4.1 ausgestaltet sind, mit dem Mittelsteg 29 des Anlageelements 26 zu verbinden. Natürlich ist es aber auch denkbar, die Flügel 27, 27.1 und 28, 28.1 kombiniert zur Aufnahme eines Gurtes, d. h. sich gegenüberliegend an den Führungen 30 zu befestigen. Damit könnte bspw. einem sich verändernden Umfang eines Ober- oder Unterschenkels eines Patienten Rechnung getragen werden, so dass sich der Tragekomfort und die Indikationsbereitschaft der modular aufgebauten Orthese 1 insgesamt durch die auswechselbaren Flügel 27, 27.1 und 28, 28.1, bzw. durch dessen Kombinationsmöglichkeit weiter verbessern lässt. Natürlich können die Flügel 27, 27.1 und 28, 28.1 auch integral mit dem als Anlageelement 26 ausgestalteten Aufnahmeelement 7, 7.1, 7.2, 7.3, 7.4, 7.5, 7.6, 7.7, 7.8, 7.9, 7.10, 7.11 ausgestaltet sein. Darüber hinaus ist es natürlich auch denkbar, die Befestigungen der Flügel 27, 27.1 und 28, 28.1 an dem Mittelsteg 29 des als Anlageelement 26 ausgestalteten Aufnahmeelementes 7, 7.1, 7.2, 7.3, 7.4, 7.5, 7.6, 7.7, 7.8, 7.9, 7.10, 7.11 durch andere kraft- und/oder formschlüssige Befestigungsmöglichkeiten auszugestalten, wie bspw. durch besondere Steckelemente, mit und ohne Einrasten, so genannte Steckpilze, Klettverschlüsse, wie beispielsweise Haken- und Flauschband (Filzband), Pilzkopfband und Veloursband Knöpfe, Pilzkopfband und Flauschband, Pilzkopfband auf Pilzkopfband oder extrudierte Haken/Pilze auf Wirkware,_Haken-Ösen-Verbindungen, Druckknöpfe, Kleben, Schrauben, Vernieten etc.. Ein weiterer Vorteil, der sich aus den an dem Mittelsteg 29 des Anlageelements 26 lösbar befestigbaren Flügel 27, 27.1 und 28, 28.1 ergibt, ist das bspw. Flügel 27, 27.1 und 28, 28.1 mit unterschiedlichen Materialeigenschaften bedarfsmäßig über das Verbindungselement 5, 5.1, 5.2 und 5.3 bzw. über die Schiene 6, 6.1, 6.2 und 6.3 mit der modular aufgebauten Orthese 1 verbindbar sind. Auch können die als Flügel 27, 27.1 und 28, 28.1 ausgestalteten Aufnahmeelemente 7, 7.1, 7.2, 7.3, 7.4, 7.5, 7.6, 7.7, 7.8, 7.9, 7.10, 7.11, an unterschiedliche Größen bzw. Formen bspw. eines Gurtes angepasst sein. Dies erhöht in vorteilhafter Weise weiterhin die Variabilität der offenbarten modular aufgebauten Orthese 1.

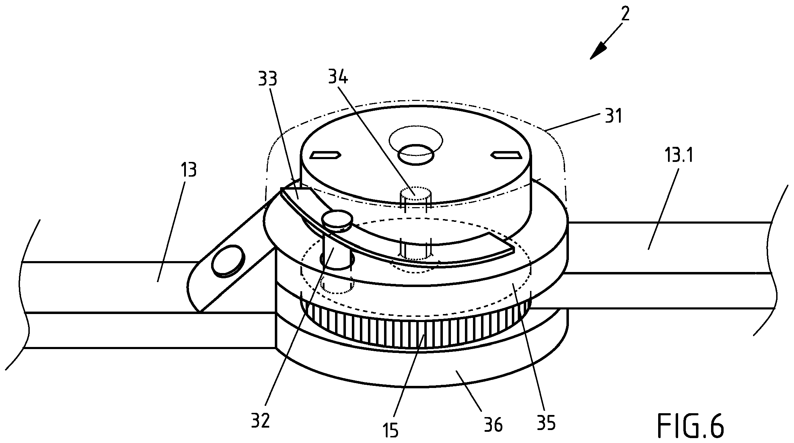

Figur 6 zeigt eine spezielle Ausführungsform des Gelenks 2 aus Figur 4 für eine Orthese 1 in einer perspektivischen Schnittansicht. Zur besseren Funktionsdarstellung des Gelenks 2 ist neben den Schenkelenden 13 und 13.1 lediglich der Gelenkeinsatz 15 des Gelenks 2 dargestellt. In vorteilhafter Weise ist das Schenkelende 13 mit zwei parallel zueinander liegenden Flächen 35 und 36 ausgestaltet, die zueinander beabstandet sind, so dass zwischen den Flächen 35 und 36 das Schenkelende 13.1 zur Aufnahme gelangt. Durch diese Darstellung soll die Funktion des in der Figur 4 dargestellten Orthesengelenks 2 nicht eingeschränkt werden. Das in der Figur 6 dargestellte Gelenk 2 weist eine Sit-/Go-Taste in Form eines Drehknopfes 31 bzw. in Form eines Druck-Drehstellers auf. Der Drehknopf 31 steht mit einem als Stift ausgestalteten Sperrelement 32, welches durch den Gelenkeinsatz 15 geführt ist, in Wirkverbindung. Das Sperrelement 32 wird dabei entlang einer auf- bzw. absteigenden Kulisse 33, die hier durch eine schraubenförmige Kontur des Drehknopfs 31 ähnlich einer Wendel ausgestaltet ist, geführt. Durch Drehung des Drehknopfs 31 wird das Sperrelement 32 der Kulisse 33 des Drehknopfs 31 folgend, hier insbesondere entlang dem Anstieg der Kulisse 33 aus dem Gelenkeinsatz 15 geführt, hier insbesondere aus dem Gelenkeinsatz 15 herausgehoben. Dadurch erfolgt eine Entsicherung des Gelenks 2 aus einer arretierten Go-Stellung in eine gelöste Sit-Stellung. Vorliegend erfolgt eine Entsicherung des Gelenks 2 durch Drehung des Drehknopfs 31 entgegen dem Uhrzeigersinn. Bei Drehung des Drehknopfs 31 mit dem Uhrzeigersinn wird das Sperrelement 32 durch den Gelenkeinsatz 15 geführt, wodurch das Gelenk 2 arretiert wird. Natürlich kann die Kulisse 33, d. h. die schraubenförmige Kulisse in dem Drehknopf 31 auch so ausgestaltet sein, dass eine Entriegelung durch den Drehknopf 31 bei Drehung mit dem Uhrzeigersinn erfolgt und eine Arretierung des Gelenks 2 durch Drehung des Drehknopfs 31 gegen den Uhrzeigersinn erfolgt. Vorliegend bildet ein durch das Schenkelende 13 geführtes Achselement 34 die Drehachse für den Drehknopf 31. Natürlich kann das Achselement 34 auch derart ausgestaltet sein, dass dieses nicht nur als Drehachse, d. h. als Drehpunkt für den Drehknopf 31 dient, sondern über das Achselement 34 der Drehknopf 31 auf dem Schenkelende 13 oder wahlweise auch auf dem Schenkelende 13.1 befestigbar ist.

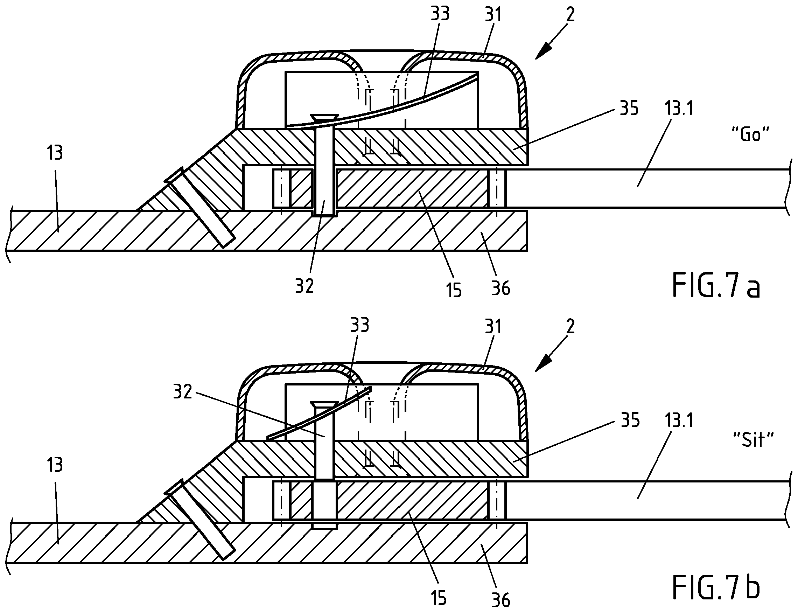

Die Figuren 7a und 7b zeigen das Gelenk 2 aus Figur 6 in einer seitlichen Schnittansicht. Dabei ist in Figur 7a die Go-Funktion des Gelenks 2 dargestellt. Wie bereits beschrieben, ist es für die Go-Funktion des Gelenks 2 notwendig, dass dieses arretiert ist. Zur Arretierung des Gelenks 2 ist das Sperrelement 32 entlang der Kulisse 33 des Drehknopfs 31 nach unten, nämlich in Richtung des Gelenkeinsatzes 15 und durch den Gelenkeinsatz 15 des Gelenks 2 geführt und durchgreift dabei das die Flächen 35 und 36 des Schenkelendes 13 und das Schenkelende 13.1 und den Gelenkeinsatz 15, was zur Arretierung des Gelenks 2 führt. In Figur 7b erfüllt das Gelenk 2, nämlich durch den Drehknopf 31 die Sit-Funktion, wobei das Gelenk 2 aus seiner Arretierung entriegelt ist. Die Entriegelung erfolgt durch Führung des Sperrelements 32 entlang der Kulisse 33, nämlich hier vorliegend entlang dem Anstieg der Kulisse 33, wodurch das Sperrelement 32 zumindest aus der Durchführung durch den Gelenkeinsatz 15 "gehoben" wird.

Insgesamt lässt sich durch den in den Figuren 7, 7a und 7b dargestellten Drehknopf 31, der anstelle des Deckelelements 16 oder auch ggf. auf das Deckelelement 16 an dem Gelenk angeordnet werden kann, die Variabilität der offenbarten modular aufgebauten Orthese 1 durch die Funktion einer Sit-/Go-Sperre erweitern, nämlich um die Funktionserweiterung einer Immobilisierungsfunktion (Geh-Funktion) und einer Entsperrung des Gelenks 2 aus der Immobilisierungsfunktion in eine Sitz-Funktion.

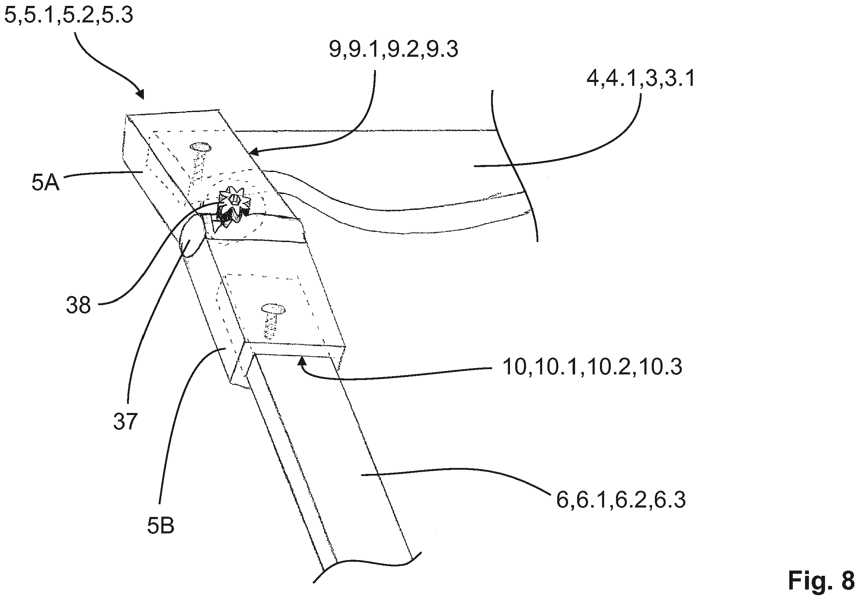

Figur 8 zeigt eine Ausführungsform eines Verbindungselements 5, 5.1, 5.2, 5.3. Das Verbindungselement 5, 5.1, 5.2, 5.3 weist einen oberen Teil 5A und einen unteren Teil 5B auf. Beide Teile 5A und 5B sind über ein Gelenk 37 miteinander verbunden. Das obere Teil 5A ist vorliegend mit einem Halteteil 4, 4.1, 3, 3.1 der modularen Orthese 1 verbunden. Das untere Teil 5B ist mit einer Schiene 6, 6.1, 6.2, 6.3 der modularen Orthese 1 verbunden. Das Gelenk 37 ist als Scharniergelenk ausgestaltet, über das die beiden Teile des Verbindungselements 5, 5.1, 5.2, 5.3 gelenkig miteinander verbunden sind. Am Gelenk 37 ist ein Einstellmechanismus ausgebildet, der mittels eines als Zahnrad ausgestalteten Einstellelements 38 bedienbar ist. Das Einstellelement 38 greift wie in der Figur 8 dargestellt mit den Zähnen des Zahnrades, die als Haltemittel bezeichnet werden können, in am Gelenk 37 ausgestaltete Gegenhaltemittel. Vorliegend weist das Einstellelement 38 eine Innensechskantaufnahme auf über die das Einstellelement 38 mittels eines Inbusschlüssels drehbar ist. Durch Drehen des Einstellelementes 38 kann das Verbindungselement 5, 5.1, 5.2, 5.3 in unterschiedliche Winkelstellungen zwischen dem oberen Teil 5A und dem unteren Teil 5B über das Gelenk 37 gebracht werden. Die Einstellung erfolgt dabei vorteilhaft stufenlos. Nach der Einstellung des Verbindungselements 5, 5.1, 5.2, 5.3 in eine definierte Winkeleinstellung kann das Gelenk 37 in der gewählten Einstellung arretiert werden. Die Einstellung kann aber auch über an die Winkelstellung angepasste Raststufen erfolgen. Durch die Einstellung der Winkelstellung des Verbindungselements 5, 5.1, 5.2, 5.3 werden auch die mit dem Verbindungselement 5, 5.1, 5.2, 5.3 verbundenen Komponenten, vorliegend die Schiene 6, 6.1, 6.2, 6.3, die mit dem unteren Teil 5B durch Einführen in die Aufnahme 10, 10.1, 10.2, 10.3 des Verbindungselements 5, 5.1, 5.2, 5.3 mit dem Verbindungselement 5, 5.1, 5.2, 5.3 verbunden ist, und das Halteteil 4, 4.1, 3, 3.1 das durch Einführen in die Ausnehmung 9, 9.1, 9.2, 9.3 in den oberen Teil 5A des Verbindungselements 5, 5.1, 5.2, 5.3 mit dem Verbindungselement 5, 5.1, 5.2, 5.3 verbunden ist, erlauben die aus diesen Komponenten ausgestaltete Orthese 1 während des Therapieverlaufes zu jedem Zeitpunkt eine Parallelität der Gelenkschienen einzustellen. Dadurch kann zum einen über das Verbindungselement 5, 5.1, 5.2, 5.3 die Orthese 1 an jede spezielle Anatomie des Knies eines Patienten angepasst und auf spezielle Indikationen einstellt werden. Zum anderen ist es erstmalig möglich eine modulare Knieorthese 1 mit einstellbarer Passform komplett aus Kunststoff herzustellen.

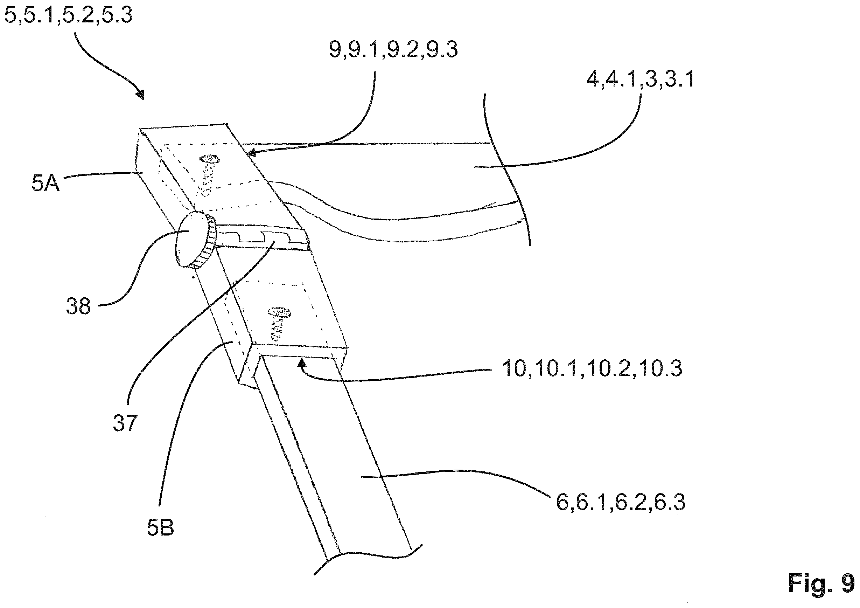

Figur 9 zeigt das Verbindungselement 5, 5.1, 5.2, 5.3 aus Figur 8, wobei jedoch das Einstellelement 38 als seitlicher Stellknopf in Form einer Rändelschraube oder Rändelmutter ausgestaltet ist, über welchen der Einstellmechanismus zur Winkeleinstellung des Verbindungselements 5, 5.1, 5.2, 5.3 über das Gelenk 37 bedienbar ist.

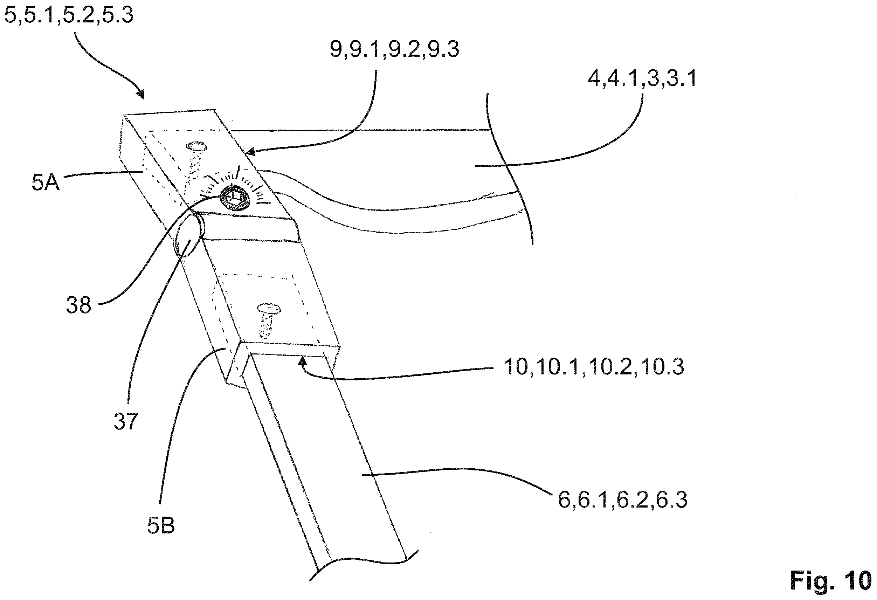

Figur 10 zeigt das Verbindungselement 5, 5.1, 5.2, 5.3 aus den vorherigen Figuren mit einem am oberen Teil 5A des Verbindungselements 5, 5.1, 5.2, 5.3 angeordneten Einstellelement 38, dass mittels eines Werkzeuges, vorliegend mit einem Inbusschlüssel bedienbar bzw. einstellbar ist. Zudem ist auf dem oberen Teil 5A des Verbindungselements 5, 5.1, 5.2, 5.3 eine Skala ausgestaltet, die zur Einstellung der Winkelstellung des zeigt das Verbindungselements 5, 5.1, 5.2, 5.3 über das Einstellelement 38 dient.

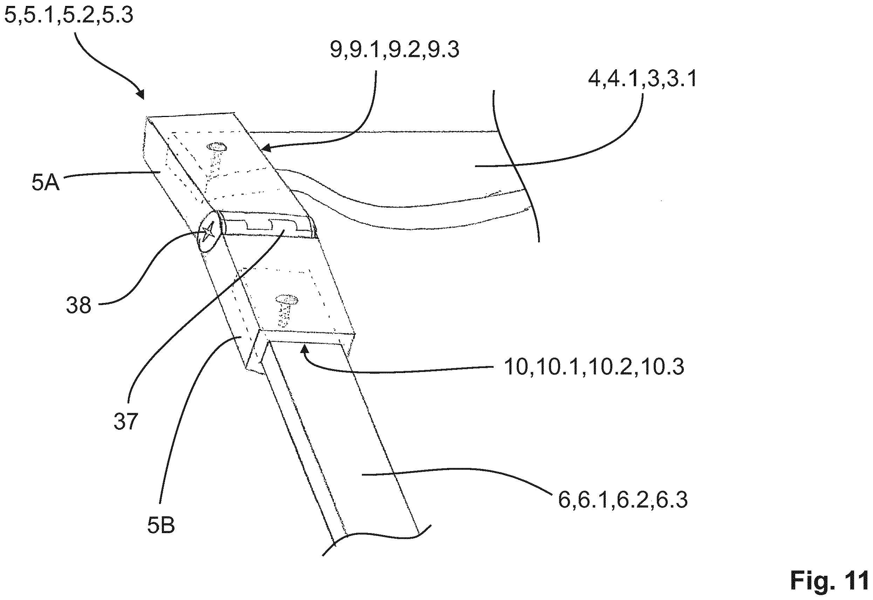

Figur 11 zeigt das Verbindungselement 5, 5.1, 5.2, 5.3 aus Figur 9, wobei das als seitlicher Stellknopf in Form einer Rändelschraube oder -mutter ausgestaltete Einstellelement 38 durch einen mittels Werkzeug bedienbaren Schraubenkopf ersetzt ist. Vorteilhaft bei dieser Ausgestaltung ist, dass das Einstellelement 38 vollständig in das Gelenk 37 eintaucht, beziehungsweise der Kopf des Einstellelements 38 nicht seitlich über das Verbindungselement 5, 5.1, 5.2, 5.3 ragt. Dadurch kann das Verbindungselement 5, 5.1, 5.2, 5.3 mit der daran angebundenen Schiene 6, 6.1, 6.2, 6.3 beispielsweise leichter in eine Schienentasche beispielsweise einer Bandage eingeführt werden.

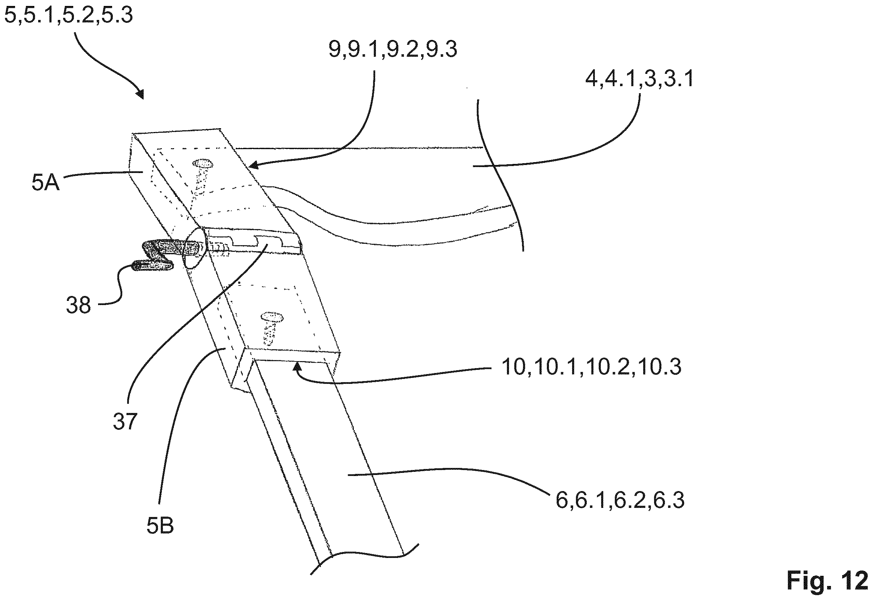

Figur 12 zeigt das Verbindungselement 5, 5.1, 5.2, 5.3 aus Figur 9 und 11, wobei der seitliche Stellknopf oder der Schraubenkopf des Einstellelements 38 durch eine Kurbel ersetzt ist. Die Kurbel kann dabei als Werkzeug in das Gelenk 37 eingeführt werden oder kann Bestandteil des Gelenkes 37 sein, wobei das als Kurbel ausgestaltete Einstellelement 38 in das Gelenk 37 versenkbar.

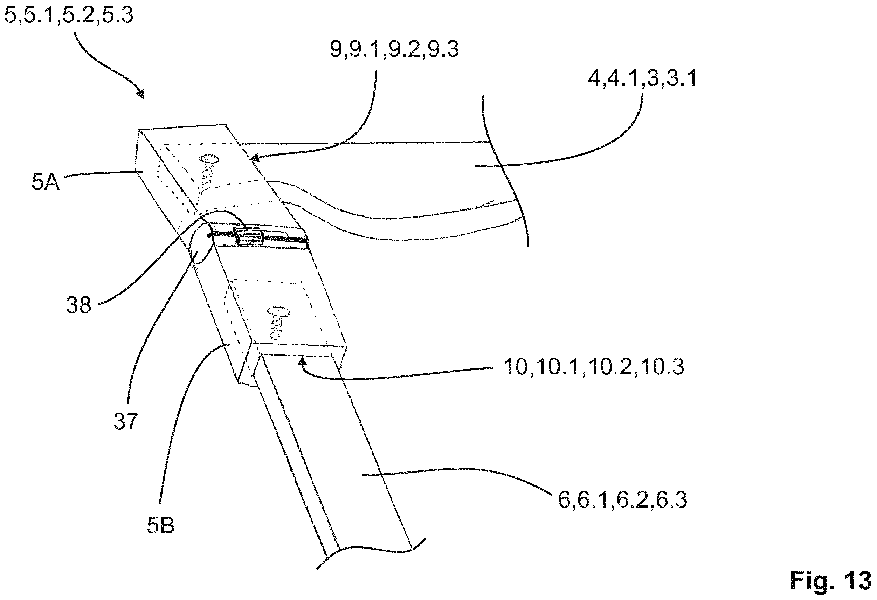

Figur 13 zeigt das Verbindungselement 5, 5.1, 5.2, 5.3 aus den vorherigen Figuren, wobei jedoch der Einstellmechanismus des Gelenks 37 mit über ein als Rändelwelle ausgestaltetes Einstellelement 38 bedienbar ist. Die Rändelwelle ist dabei sowohl um die Achse des Gelenks 37 drehbar als auch vorteilhaft stufenlos in axialer Längserstreckung des Gelenks 37 verschiebbar.

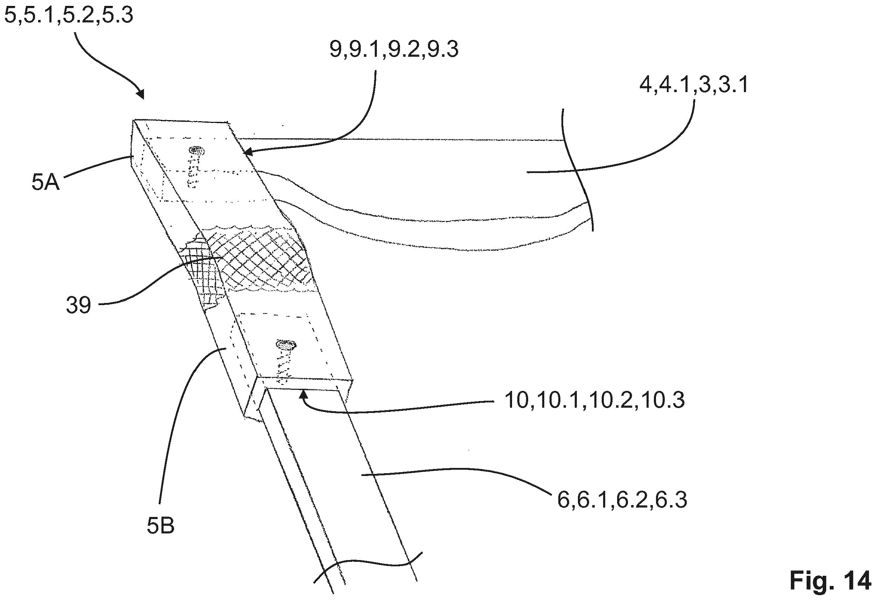

Figur 14 zeigt eine weitere Ausführungsform eines Verbindungselements 5, 5.1, 5.2, 5.3 mit flexiblen Mittelteil 39, über das der obere Teil 5A mit dem unteren Teil 5B des Verbindungselements 5, 5.1, 5.2, 5.3 miteinander verbunden sind. Vorliegend ist das flexible Mittelteil 39 einstückig mit dem oberen Teil 5A und dem unteren Teil 5B des Verbindungselements 5, 5.1, 5.2, 5.3 ausgestaltet. Durch das flexible Mittelteil 39 ist der obere Teil 5A relativ zu dem unteren Teil 5B in alle Richtungen verdrehbar bzw. ist der obere Teil 5A relativ zu dem unteren Teil 5B verdrillbar. Diese Ausgestaltung des Verbindungselements 5, 5.1, 5.2, 5.3 ermöglicht ein Winkeleinstellung bei gleichzeitiger Verdrehung des oberen Teils 5A relativ zu dem unteren Teil 5B und erweitert dadurch die Einstellmöglichkeiten der Parallelstellung der Schienen.

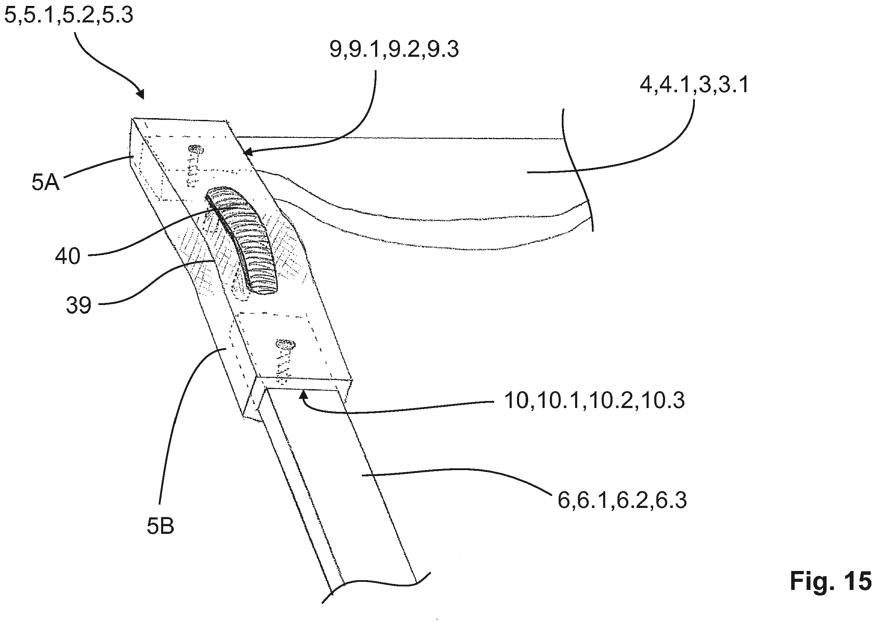

Figur 15 zeigt das Verbindungselement 5, 5.1, 5.2, 5.3 mit flexiblen Mittelteil 39 aus Figur 14 mit einem auf das flexible Mittelteil 39 aufgesetzten Fixier-Clip 40. Der Fixier-Clip 40 ist in unterschiedlichen Winkeleinstellungen ausgestaltet und gibt so die gewünschte Winkelstellung des Verbindungselements 5, 5.1, 5.2, 5.3 beim Aufclipsen über das flexible Mittelteil 39 vor.

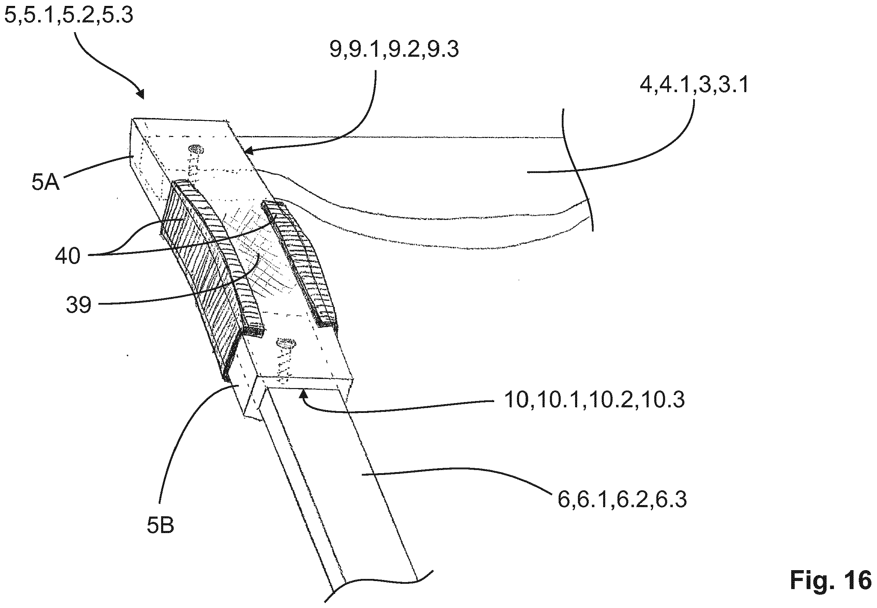

Figur 16 zeigt das Verbindungselement 5, 5.1, 5.2, 5.3 mit flexiblen Mittelteil 39 aus Figur 14 mit seitlich an das flexible Mittelteil 39 aufgesetzten Fixier-Clips 40 mit voreingestellter Winkeleinstellung.

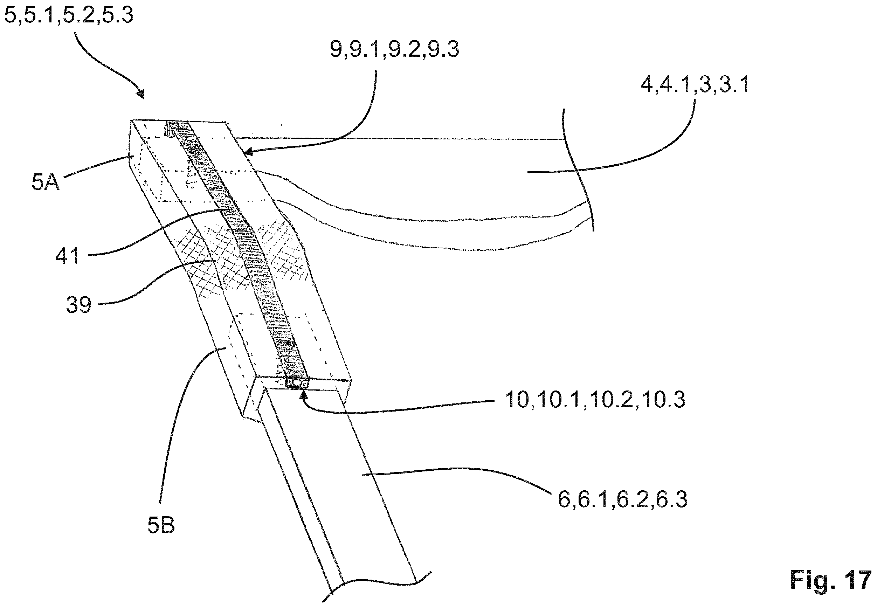

Figur 17 zeigt das Verbindungselement 5, 5.1, 5.2, 5.3 mit flexiblen Mittelteil 39 aus Figur 14 mit einer über das flexible Mittelteil 39 übergreifenden Winkelspange 41, die die gleiche Funktion wie die Fixier-Clips 40 erfüllt. Sowohl die Winkelspange 41 als auch die Fixier-Clips 40 können aus Metall oder Kunststoff oder aus einer Kombination dieser Materialien hergestellt sein.

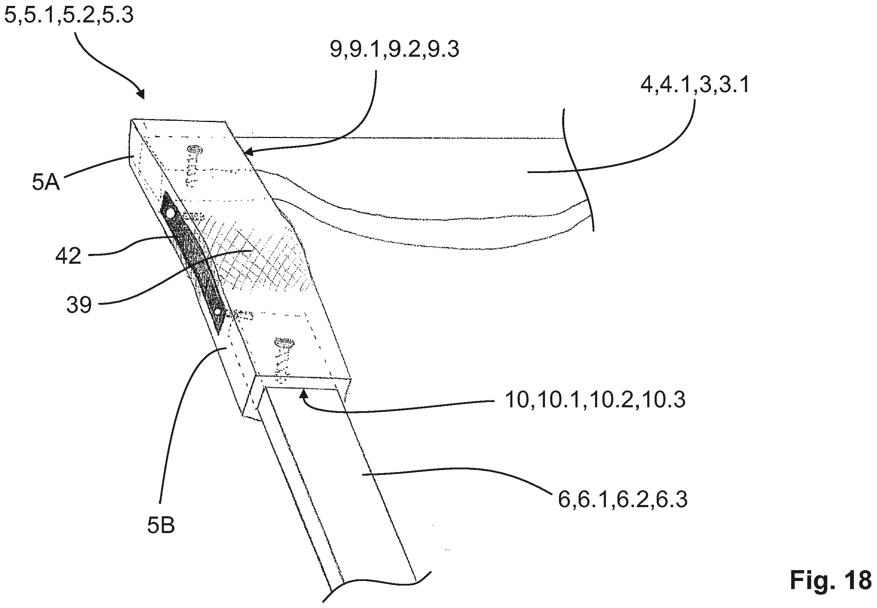

Figur 18 zeigt das Verbindungselement 5, 5.1, 5.2, 5.3 mit flexiblen Mittelteil 39 aus Figur 14 mit einem seitlich auf-/eingesetzten Stabilisator 42, der die gleiche Funktion wie die Fixier-Clips 40 oder die Winkelspange 41 erfüllt.

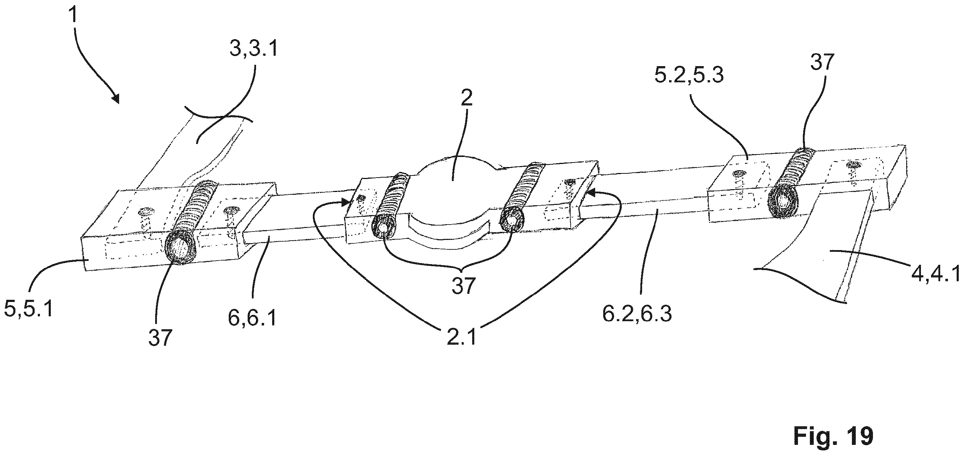

Figur 19 zeigt zwei Verbindungselemente 5, 5.1 und 5.2, 5.3 mit Gelenk 37, welche jeweils mit einem Schienenteil 6, 6.1 und 6.2, 6.3 einer Gelenkschiene verbunden sind, wobei das Orthesengelenk 2 der Gelenkschiene zwei Gelenke 37, die als Scharniergelenke ausgestaltet sind, aufweist. Die als Scharniergelenke ausgestalteten Gelenke 37 des Orthesengelenks 2 sind vorteilhaft wie die Gelenke 37 des Verbindungselements 5, 5.1, 5.2, 5.3 ausgestaltet und bedienbar. Insgesamt werden durch die zusätzliche gelenkige Ausgestaltung des Orthesengelenks 2 die Einstellmöglichkeiten der Orthese 1 an die anatomischen Gegebenheiten eines Patienten und im Verlauf der Therapie noch erweitert.

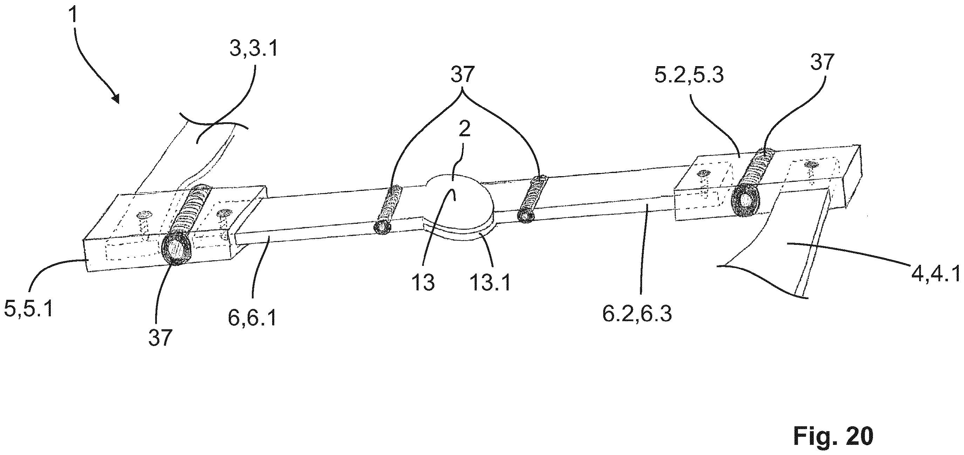

Figur 20 zeigt zwei Verbindungselemente 5, 5.1 und 5.2, 5.3 mit Gelenk 37, welche jeweils mit einem Schienenteil 6, 6.1 und 6.2, 6.3 einer Gelenkschiene verbunden sind, wobei das Orthesengelenk 2 der Gelenkschiene über die Schenkelenden 13, 13.1 der Schienen 6, 6.1 und 6.2, 6.3 gebildet wird. Zwischen den Schenkelenden 13, 13.1 und den Teilen der Schienen 6, 6.1 und 6.2, 6.3, welche mit den Verbindungselementen 5, 5.1 und 5.2, 5.3 verbunden ist, ist jeweils ein Gelenk 37 ausgestaltet. Die als Scharniergelenke ausgestalteten Gelenke 37 im Anschluss an die Schenkelenden 13, 13.1 sind vorteilhaft wie die Gelenke 37 des Verbindungselements 5, 5.1, 5.2, 5.3 ausgestaltet und bedienbar. Insgesamt werden durch die zusätzliche gelenkige Ausgestaltung der Schienen 6, 6.1 und 6.2, 6.3 durch jeweils ein Gelenk 37 im Anschluss an die Schenkelenden 13, 13.1 die Einstellmöglichkeiten der Orthese 1 an die anatomischen Gegebenheiten eines Patienten und im Verlauf der Therapie noch erweitert.

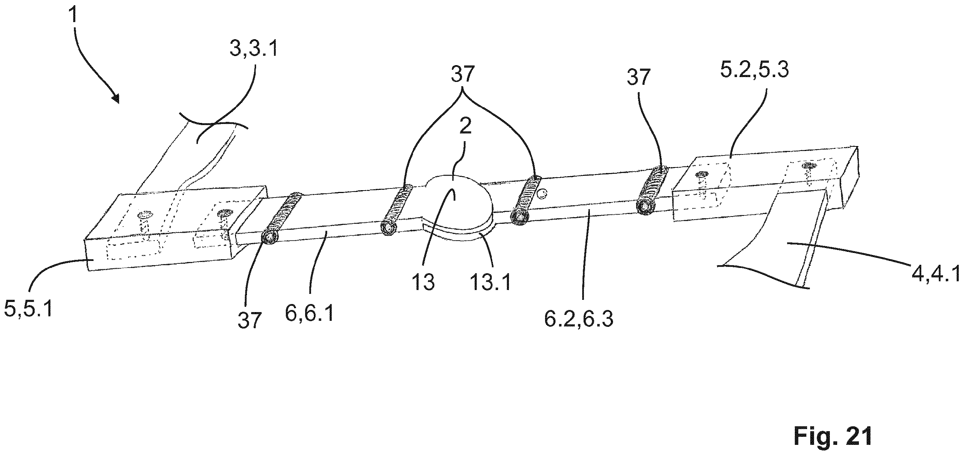

Figur 21 zeigt zwei Verbindungselemente 5, 5.1 und 5.2, 5.3, welche jeweils mit einem Schienenteil 6, 6.1 und 6.2, 6.3 einer Gelenkschiene verbunden sind, wobei das Orthesengelenk 2 der Gelenkschiene über die Schenkelenden 13, 13.1 der Schienen 6, 6.1 und 6.2, 6.3 gebildet wird. Zwischen den Schenkelenden 13, 13.1 und den Teilen der Schienen 6, 6.1 und 6.2, 6.3, welche mit den Verbindungselementen 5, 5.1 und 5.2, 5.3 verbunden ist, sind jeweils zwei Gelenke 37 ausgestaltet. Die als Scharniergelenke ausgestalteten Gelenke 37 im Anschluss an die Schenkelenden 13, 13.1 und die im Anschluss an die Verbindungselemente 5, 5.1 und 5.2, 5.3 in den Schienen 6, 6.1 und 6.2, 6.3 ausgestalteten Gelenke 37 sind vorteilhaft wie die Gelenke 37 des Verbindungselements 5, 5.1, 5.2, 5.3 ausgestaltet und bedienbar. Insgesamt werden durch die zusätzliche gelenkige Ausgestaltung der Schienen 6, 6.1 und 6.2, 6.3 durch jeweils ein Gelenk 37 im Anschluss an die Schenkelenden 13, 13.1 und im Anschluss an die Verbindungselemente 5, 5.1 und 5.2, 5.3 die Einstellmöglichkeiten der Orthese 1 an die anatomischen Gegebenheiten eines Patienten und im Verlauf der Therapie erweitert, ohne dass die Verbindungselemente 5, 5.1 und 5.2, 5.3 selber ein Gelenk 37 aufweisen müssen.

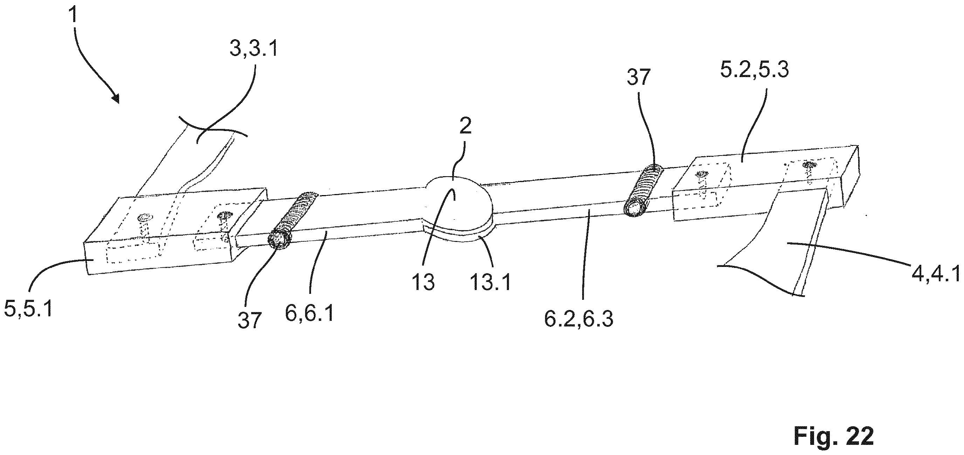

Figur 22 zeigt die Gelenkschiene aus Figur 21 mit nur jeweils einem als Scharniergelenk im Anschluss an die Verbindungselemente 5, 5.1 und 5.2, 5.3 in den Schienen 6, 6.1 und 6.2, 6.3 ausgestalteten Gelenk 37. Gegenüber der Gelenkschiene aus Figur 21 sind die Einstellmöglichkeiten der damit ausgebildeten Orthese 1 an die anatomischen Gegebenheiten eines Patienten und im Verlauf der Therapie etwas verringert. Jedoch kann diese Gelenkschiene, die weiterhin zusätzliche Einstellmöglichkeiten gegenüber den bekannten Gelenkschienen ermöglicht mit geringeren Kosten gegenüber der in Figur 21 dargestellten Scheine hergestellt werden, da auf insgesamt zwei Gelenke 37 verzichtet wird.

Insgesamt können die in der Schiene 6, 6.1, 6.2, 6.3 und/oder die in dem Orthesengelenk ausgestalteten Gelenke 37 auch durch das für das Verbindungselement 5, 5.1, 5.2, 5.3 beschriebene flexible Mittelteil 38 ersetzt werden. Zur Arretierung einer Winkelstellung oder zur Einstellung einer Winkelstellung über das flexible Mittelteil 38 dienen dann die für das Verbindungselement 5, 5.1, 5.2, 5.3 beschriebenen Fixier-Clipse 40, Winkelspangen 41 und/oder Stabilisatoren 42.

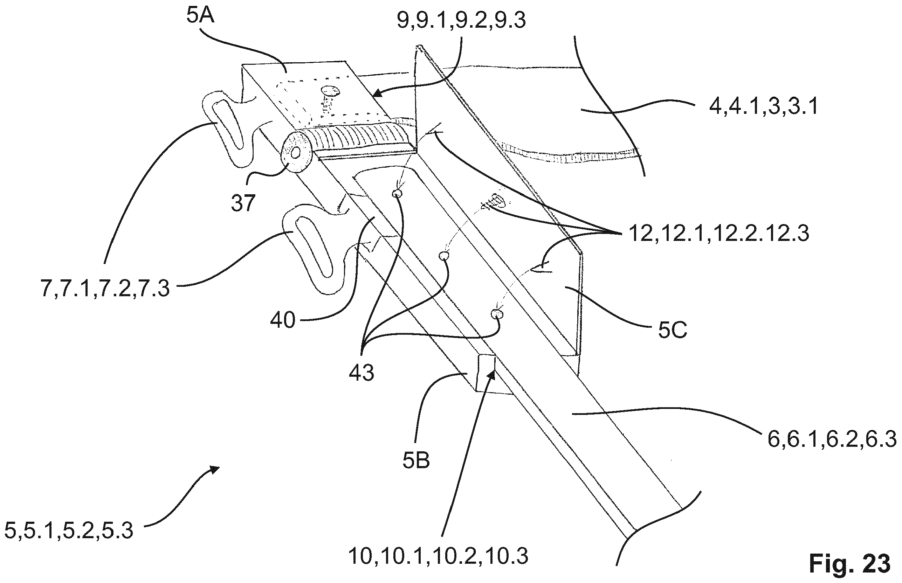

Figur 23 eine alternative Ausführungsform eines Verbindungselements 5, 5.1, 5.2, 5.3, das zum Einführen der Schiene 6, 6.1 oder 6.2, 6.3 in die Aufnahme 10, 10.1, 10.2, 10.3 über einen Deckel 5C geöffnet werden kann. Der Deckel 5 erstreckt axial über die gesamte Länge der Aufnahme 10, 10.1, 10.2, 10.3 des Verbindungselement 5, 5.1, 5.2, 5.3 und gibt beim Öffnen senkrecht zur Längserstreckung der Aufnahme 10, 10.1, 10.2, 10.3 die Aufnahme 10, 10.1, 10.2, 10.3 zum Einführen bzw. zum Einlegen der Schiene 6, 6.1 oder 6.2 in die Aufnahme frei. Durch Öffnen der Aufnahme 10, 10.1, 10.2, 10.3 über den Deckel 5C kann zudem ein Aufnahmeelement 7, 7.1..., hier das in der Figur untere Aufnahmeelement 7, 7.1... variabel in Längserstreckung der Aufnahme über einen damit einstückig ausgestalteten Fixier-Clip 40 mit dem Verbindungselement 5, 5.1, 5.2, 5.3 verbunden werden, wobei das Aufnahmeelement 7, 7.1... durch Schließen des Deckels 5C über den Fixier-Clip 40 in seiner Position und am Verbindungselement 5, 5.1, 5.2, 5.3 festgelegt wird. Natürlich kann das Aufnahmeelement 7, 7.1... auch einstückig mit dem vorhergenannten Fixier-Clip 40, der zur Arretierung der Winkelstellung des Verbindungselements 5, 5.1, 5.2, 5.3 mit flexiblem Mittelteil 39 dient, ausgestaltet sein. Zum kraftschlüssigen Verbindung des Deckels 5A an das Verbindungselement 5, 5.1, 5.2, 5.3 dienen Befestigungselemente 12, 12.1, 12.2, 12.3 vorliegend in Form von Schraubverbindungen, die durch den Deckel 5A und durch Durchführungen 43 der Schiene 6, 6.1, 6.2, 6.3 greifen. Entgegen der Darstellung kann der Deckel 5A auch vorne, hinten oder seitlich am Verbindungselement 5, 5.1, 5.2, 5.3 ausgestaltet sein. Anstelle des hier dargestellten aufklappbaren Deckels 5A kann der Deckel 5A auch auf das Verbindungselement 5, 5.1, 5.2, 5.3 aufgeschoben, aufgeschraubt oder aufgesteckt werden.

Anstelle der hier als Schraubverbindungen dargestellten Befestigungselemente 12, 12.1, 12.2, 12.3 können auch beispielsweise Einsteckelemente oder Clipsverbindungen dienen, welche vorteilhafterweise eine zumindest kraftschlüssige Kopplung des Deckels 5A an dem Verbindungselement 5, 5.1, 5.2, 5.3, wobei die kraftschlüssige Kopplung des Deckels 5A über die Befestigungselemente 12, 12.1, 12.2, 12.3 wieder lösbar ist, ohne dass dabei vorzugsweise die Befestigungselemente 12, 12.1, 12.2, 12.3 und der Deckel 5A zerstört werden.

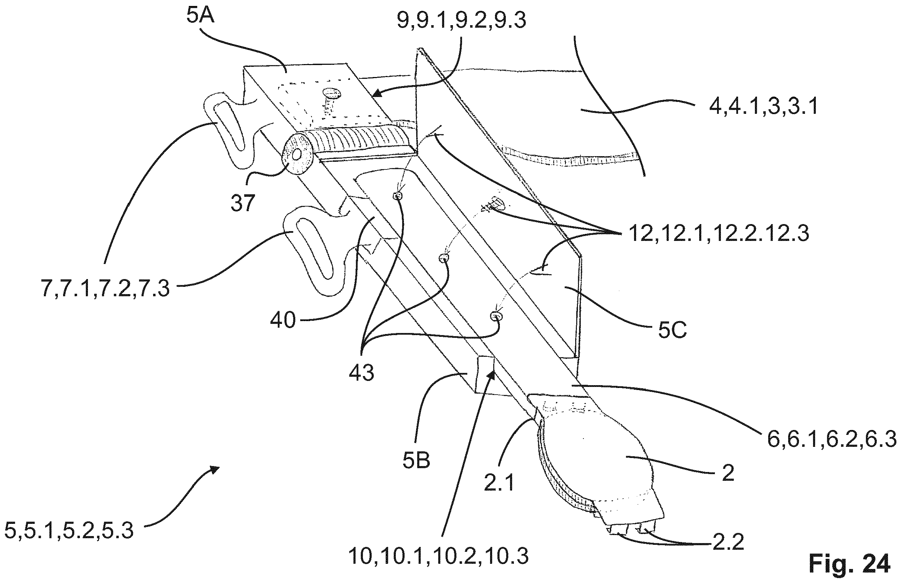

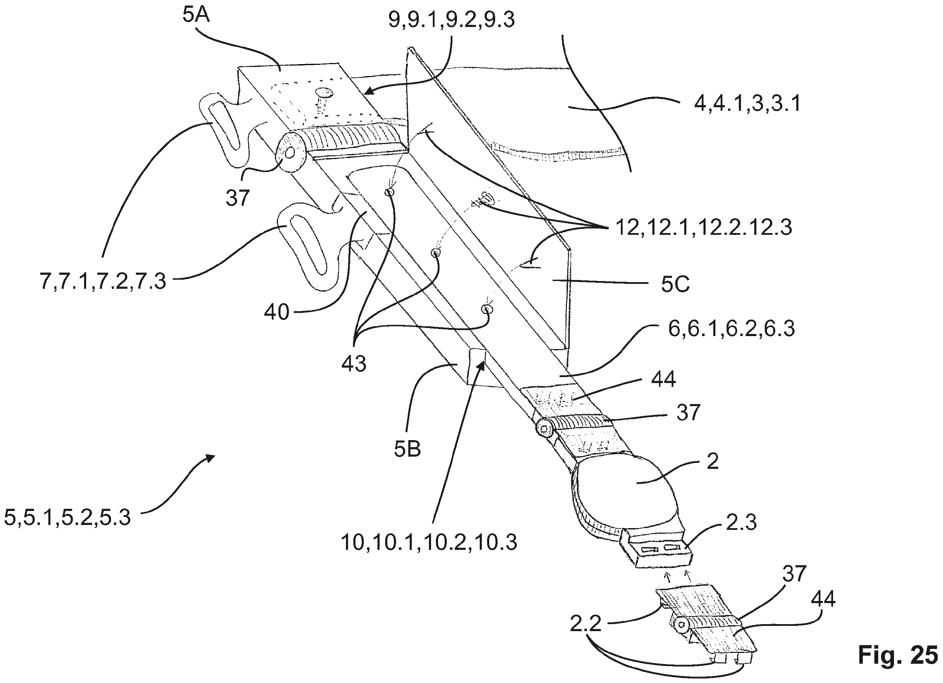

Figur 24 zeigt das Verbindungselement 5, 5.1, 5.2, 5.3 aus Figur 23 mit einem an die in die Aufnahme 10, 10.1, 10.2, 10.3 eingelegte Schiene 6, 6.1, 6.2, 6.3 angeklipsten Orthesengelenk 2. Die Schiene 6, 6.1, 6.2, 6.3 weist dazu eine hier nicht im Detail dargestellte Aufnahme 2.1 auf, in die das Orthesengelenk 2 mit daran ausgebildeten Clipsen 2.2 greift (Die Aufnahme 2.1 an der Schiene 6, 6.1, 6.2, 6.3 ist gleich der in Figur 25 dargestellten Aufnahme 2.3 des Orthesengelenks 2 für ein daran anzuklipsendes Zwischenelement 44 ausgestaltet). Anstelle des Orthesengelenks 2 kann an die Aufnahme 2.1 der Schiene 6, 6.1, 6.2, 6.3 auch eine andere Komponente oder ein Zusatzmodul, wie beispielsweise ein die Schiene 6, 6.1, 6.2, 6.3 verlängerndes Schienenteil angeklipst werden. Ein Beispiel für ein Zusatzmodul, das an die Aufnahme 2.1 der Schiene 6, 6.1, 6.2, 6.3 anklipsbar ist, ist in Figur 25 gezeigt.

Figur 25 zeigt das Verbindungselement 5, 5.1, 5.2, 5.3 aus Figur 24 mit einem zwischen dem Orthesengelenk 2 und der Schiene 6, 6.1, 6.2, 6.3 eingesetzten Zwischenelement 44 mit als Scharniergelenk ausgestaltetem Gelenk 37. Das Gelenk 37 kann wie für das Gelenk 37 des Verbindungselementes 5, 5.1, 5.2, 5.3 und wie für das Gelenk 37 der Schiene 6, 6.1, 6.2, 6.3 und des Orthesengelenks 2 beschrieben, ausgestaltet sein und die gleichen Funktionen erfüllen. Zum Anschluss des Zwischenelements 44 an das Orthesengelenk 2 weist das Orthesengelenk 2 eine Aufnahme 2.3 auf in die das Zwischenelement 44 mit daran ausgestalteten Clipsen 2.2 greift.

Ganz allgemein können die Clipse 2.2 als Haltemittel und die Aufnahmen 2.1 und 2.3 als Gegenhaltemittel verstanden werden, wobei die Haltemittel zumindest formschlüssig und/oder kraftschlüssig mit den Gegenhaltemitteln koppelbar sind. Insofern können die Clipse 2.2 und die Aufnahmen 2.1 und 2.3 durch andere Halte- bzw. Gegenhaltemittel erstzt werden die miteinander formschlüssig und/oder kraftschlüssig koppelbar sind.

### Bezugszeichenliste

- 1: Orthese
- 2: Orthesengelenk
- 2.1: Aufnahme zu 2 für Schiene
- 2.2: Clipse für Clipsverbindung
- 2.3: Aufnahme für Zwischenelement
- 3: Halteteil
- 3.1: Halteteil
- 4: zweites Halteteil
- 4.1: zweites Halteteil
- 5: Verbindungselement
- 5.1: Verbindungselement
- 5.2: Verbindungselement
- 5.3: Verbindungselement
- 5A: ober Teil / zweiteiliges Verbindungselement
- 5B: unterer Teil / zweiteiliges Verbindungselement
- 5C: Deckel von Verbindungselement
- 6: Schiene/Schienenschenkel
- 6.1: Schiene/Schienenschenkel
- 6.2: Schiene/Schienenschenkel
- 6.3: Schiene/Schienenschenkel
- 7: Aufnahmeelement
- 7.1: Aufnahmeelement
- 7.2: Aufnahmeelement
- 7.3: Aufnahmeelement
- 7.4: Aufnahmeelement
- 7.5: Aufnahmeelement
- 7.6: Aufnahmeelement
- 7.7: Aufnahmeelement
- 7.8: Aufnahmeelement
- 7.9: Aufnahmeelement
- 7.10: Aufnahmeelement
- 7.11: Aufnahmeelement
- 8: Ausnehmung (Langloch)
- 8.1: Ausnehmung (Langloch)
- 8.2: Ausnehmung (Langloch)
- 8.3: Ausnehmung (Langloch)
- 9: Ausnehmung
- 9.1: Ausnehmung
- 9.2: Ausnehmung
- 9.3: Ausnehmung
- 10: Aufnahme
- 10.1: Aufnahme
- 10.2: Aufnahme
- 10.3: Aufnahme
- 12: Befestigungselement
- 12.1: Befestigungselement
- 12.2: Befestigungselement
- 12.3: Befestigungselement
- 13: Schenkelende
- 13.1: Schenkelende
- 14: Lagerausnehmung
- 15: Gelenkeinsatz
- 15.1: Gelenkeinsatz (Kreisscheibe)
- 16: Deckelelement
- 17: Markierung
- 19: Anschlag
- 20: Teil der Kreisscheibe 15.1
- 20.1: Teil der Kreisscheibe 15.1
- 21: Zapfen
- 22: Aussparung
- 23: weiteres Bauteil
- 24: Befestigungselement
- 25: Lagerscheibe
- 26: Anlageelement
- 27, 27.1: Flügel/ Flügelpaar
- 28, 28.1: Flügel/ Flügelpaar
- 29: Mittelsteg
- 30: Führung
- 31: Drehknopf
- 32: Sperrelement
- 33: Kulisse
- 34: Achselement
- 35: Fläche zu Schenkelende 13
- 36: Fläche zu Schenkelende 13
- 37: Gelenk/ Scharniergelenk
- 38: Einstellelement für Einstellmechanismus
- 39: flexibles Mittelteil im Verbindungselement
- 40: Fixier-Clip
- 41: Winkelspange
- 42: Stabilisator
- 43: Durchführungen in Schiene
- 44: Zwischenelement

## Patentansprüche

1. Modular aufgebaute Orthese (1), insbesondere eine Knieorthese, umfassend folgende mechanisch zusammenfügbare Komponenten:
- mindestens ein Orthesengelenk (2),
- ein erstes Halteteil (3),
- ein zweites Halteteil (4),
- mindestens ein Verbindungselement (5, 5.1, 5.2, 5.3),
- mindestens eine Schiene (6, 6.1, 6.2, 6.3),
wobei die Halteteile (3, 4) jeweils über ein Verbindungselement (5, 5.1, 5.2, 5.3) an oder auf jeweils einer Schiene (6, 6.1, 6.2, 6.3) befestigbar sind, und wobei wenigstens zwei Schienen (6, 6.2 oder 6.1, 6.3) das erste und das zweite Halteteil (3, 4) mit dem Orthesengelenk (2) verbinden,
wobei das Halteteil eine Spange (3.1, 4.1) oder ein Gurt ist, wobei der Gurt und/oder die Spange (3.1, 4.1) über das Verbindungselement (5, 5.1, 5.2, 5.3) mit der Schiene (6, 6.1, 6.2, 6.3) verbindbar ist, wodurch eine Gelenkführungsorthese ohne oder mit Extensions-/Flexionsbegrenzung entsteht, **dadurch gekennzeichnet dass** in der Spange (3.1, 4.1) und/oder dem Gurt Ausnehmungen (8, 8.1, 8.2, 8.3) zum Justieren und zur Weitenverstellung der Spange (3.1, 4.1) und/oder des Gurtes an oder in den Verbindungselementen (5, 5.1, 5.2, 5.3) ausgestaltet sind, wobei das Halteteil (3) und das Halteteil (4) als Oberschenkelspange oder Oberschenkelgurt (3.1) und als Unterschenkelspange oder Unterschenkelgurt (4.1) gegenläufig zueinander nämlich als vordere Spange oder Gurt (3.1) und als hintere Spange oder Gurt (4.1) befestigt sind, wobei das Verbindungselement (5, 5.1, 5.2, 5.3) Befestigungselemente (12, 12.2) zum Befestigen von Aufnahmeelementen (7, 7.1, 7.2, 7.3, 7.4, 7.5, 7.6, 7.7, 7.8, 7.9, 7.10, 7.11) umfasst, wobei sowohl das als Oberschenkelspange oder Oberschenkelgurt (3.1) ausgestaltete Halteteil (3) als auch das als Unterschenkelspange oder Unterschenkelgurt (4.1) ausgestaltete Halteteil (4) durch Lösen der Befestigungselemente (12, 12.1, 12.2, 12.3) lösbar und durch eine andere Spange oder Gurt (3.1, 4.1), die der Größe des Ober-und/oder des Unterschenkels eines Patienten angepasst ist, austauschbar ist.

2. Modular aufgebaute Orthese (1) nach Anspruch 1,
**dadurch gekennzeichnet,**
**dass** das Verbindungselement (5, 5.1, 5.2, 5.3) als ein ein- oder mehrteiliges Scharniergelenk ausgestaltet ist und/oder die Schiene (6, 6.1, 6.2, 6.3) ein ein- oder mehrteiliges Scharniergelenk aufweist.

3. Modular aufgebaute Orthese (1) nach einem der vorhergehenden Ansprüche,
**dadurch gekennzeichnet,**
**dass** das Verbindungselement (5, 5.1, 5.2, 5.3) wenigstens eine Aufnahme (10, 10.1, 10.2, 10.3) für die Schiene (6, 6.1, 6.2, 6.3) aufweist, wobei die Schiene (6, 6.1, 6.2, 6.3) kraft- und/oder formschlüssig über die Aufnahme (10, 10.1, 10.2, 10.3) mit dem Halteteil (3, 4) verbunden ist.

4. Modular aufgebaute Orthese (1) nach Anspruch 3,
**dadurch gekennzeichnet,**
**dass** die kraft- und/oder formschlüssige Verbindung zwischen dem Halteteil (3, 4) und der Schiene (6, 6.1, 6.2, 6.3) höhenvariabel über die Aufnahme (10, 10.1, 10.2, 10.3) und/oder das Halteteil (3, 4) einstellbar ist.

5. Modular aufgebaute Orthese (1) nach einem der vorhergehenden Ansprüche,
**dadurch gekennzeichnet,**
**dass** das Verbindungselement (5, 5.1, 5.2, 5.3) Befestigungselemente (12, 12.2) zum Befestigen von Gurtaufnahmeelementen umfasst.

6. Modular aufgebaute Orthese (1) nach einem der vorhergehenden Ansprüche, zum Einführen in eine Schienentasche einer Bandage, wobei insbesondere eine in der Schienentasche aufgenommene Schiene (6, 6.1, 6.2, 6.3) endseitig verlängerbar ist.

7. Modular aufgebaute Orthese (1) nach einem der vorhergehenden Ansprüche,
**dadurch gekennzeichnet,**
**dass** das Verbindungselement (5, 5.1, 5.2, 5.3) zur Ausgestaltung zur wahlweisen Befestigung eines oberen oder unteren Halteteils (3, 4) und/oder zur gleich- und/oder gegenläufigen Anlage von Halteteilen (3, 4) an der Schiene (6, 6.1, 6.2, 6.3) symmetrisch ausgestaltet ist.

8. Modular aufgebaute Orthese (1) nach einem der vorhergehenden Ansprüche,
**dadurch gekennzeichnet,**
**dass** die Spange (3.1, 4.1) einteilig zum Einsetzen in die Ausnehmungen (9, 9.1, 9.2, 9.3) des Verbindungselements (5, 5.1, 5.2, 5.3) und/oder zum Durchführen durch die Ausnehmungen (9, 9.1, 9.2, 9.3) des Verbindungselements (5, 5.1, 5.2, 5.3) ausgestaltet ist.

9. Modular aufgebaute Orthese (1) nach einem der vorhergehenden Ansprüche,
**dadurch gekennzeichnet,**
**dass** an der Schiene (6, 6.1, 6.2, 6.3) Befestigungselemente (12, 12.2) zum Befestigen von Aufnahmeelementen (7, 7.1, 7.2, 7.3, 7.4, 7.5, 7.6, 7.7, 7.8, 7.9, 7.10, 7.11), insbesondere von Gurtaufnahmeelementen, ausgestaltet sind.

10. Modular aufgebaute Orthese (1) nach einem der vorhergehenden Ansprüche,
**dadurch gekennzeichnet,**
**dass** die Schiene (6, 6.1, 6.2, 6.3) konvex und/oder konkav ausgestaltete Bereiche aufweist, die einen festen Sitz der Orthese (1) am Patienten unterstützen.

11. Modular aufgebaute Orthese (1) nach einem der vorhergehenden Ansprüche,
**dadurch gekennzeichnet,**
**dass** das Aufnahmeelement (7, 7.1, 7.2, 7.3, 7.4, 7.5, 7.6, 7.7, 7.8, 7.9, 7.10, 7.11) kraft- und/oder formschlüssig mit dem Verbindungselement (5, 5.1, 5.2, 5.3) und/oder der Schiene (6, 6.1, 6.2, 6.3) verbunden ist.

12. Modular aufgebaute Orthese (1) nach einem der vorhergehenden Ansprüche mit einem Gelenk (2),
wobei das Gelenk (2) wenigstens zwei Schienen (6, 6.2 oder 6.1, 6.3) verbindet, insbesondere Schienen (6, 6.1, 6.2, 6.3) nach einem der Ansprüche 1 bis 4, und 6 bis 7 und 10 bis 12, und über ein Verbindungselement (5, 5.1, 5.2, 5.3), insbesondere ein Verbindungselement (5, 5.1, 5.2, 5.3) nach einem der Ansprüche 1 bis 5, 7 bis 9 und 12, mit den Schienen (6, 6.1, 6.2, 6.3) kraft- und/oder formschlüssig verbundene Halteteile (3, 4), insbesondere Halteteile (3, 4) nach einem der Ansprüche 1, 3, 4, 7 und 8, verbindet, wobei zwei mit den Schienen (6, 6.1, 6.2, 6.3) verbundene Schenkelteile, deren aufeinander zugerichtete Schenkelenden (13, 13.1) Bestandteile der Schienen (6, 6.1, 6.2, 6.3) und/oder des Gelenks (2) sind, über eine Lagerausnehmung (14), vorzugsweise eine Führungskulisse und einen die Lagerausnehmung (14) eingreifender Gelenkeinsatz (15) schwenkbar miteinander verbunden sind.

13. Modular aufgebaute Orthese (1) nach Anspruch 12,
**dadurch gekennzeichnet,**
**dass** der Gelenkeinsatz (15) an der zugehörigen Schiene (6, 6.1, 6.2, 6.3) in unterschiedlichen Drehstellungen feststellbar ist.

14. Modular aufgebaute Orthese nach Anspruch 12 oder 13,
**dadurch gekennzeichnet,**
**dass** der Gelenkeinsatz (15) als Kreisscheibe (15.1) mit einer Umfangsverzahnung ausgebildet ist, und dass die zugehörige Schiene (6, 6.1, 6.2, 6.3) ein mit einer der Umfangsverzahnung entsprechenden Innenverzahnung versehene Lagerausnehmung (14) zur Aufnahme des Gelenkeinsatzes aufweist.

15. Modular aufgebaute Orthese (1) nach einem der Ansprüche 12 bis 14,
**dadurch gekennzeichnet,**
**dass** die für den Gelenkeinsatz (15, 15.1) vorgesehene Lagerausnehmung (14) als Führungskulisse den gegenseitigen Drehwinkel der Schienen (6, 6.1, 6.2, 6.3) begrenzende Anschläge bildet.

## Claims

1. Modularly structured orthosis (1), in particular a knee orthosis, comprising the following mechanically joinable components:
- at least one orthotic joint (2),
- a first holding part (3),
- a second holding part (4),
- at least one connecting element (5, 5.1, 5.2, 5.3),
- at least one rail (6, 6.1, 6.2, 6.3),
wherein the holding parts (3, 4) can each be fastened via a connecting element (5, 5.1, 5.2, 5.3) to or on a respective rail (6, 6.1, 6.2, 6.3), and wherein at least two rails (6, 6.2 or 6.1, 6.3) connect the first and second holding parts (3, 4) to the orthotic joint (2), wherein the holding part is a clasp (3.1, 4.1) or a strap,
wherein the strap and/or the clasp (3.1, 4.1) can be connected to the rail (6, 6.1, 6.2, 6.3) via the connecting element (5, 5.1, 5.2, 5.3), thereby creating a joint guiding orthosis with no or with extension/flexion limitation,
**characterised in that** recesses (8, 8.1, 8.2, 8.3) are formed in the clasp (3.1, 4.1) and/or the strap for adjusting and for width adjustment of the clasp (3.1, 4.1) and/or the strap at or in the connecting elements (5, 5.1, 5.2, 5.3),
wherein the holding part (3) and the holding part (4) are fastened as a thigh clasp or thigh strap (3.1) and as a lower-leg clasp or lower-leg strap (4.1) in opposite directions to one another, namely as a front clap or strap (3.1) and as a rear clasp or strap (4.1),
wherein the connecting element (5, 5.1, 5.2, 5.3) comprises fastening elements (12, 12.2) for fastening receiving elements (7, 7.1, 7.2, 7.3, 7.4, 7.5, 7.6, 7.7, 7.8, 7.9, 7.10, 7.11) wherein both the holding part (3) designed as a thigh clasp or thigh strap (3.1) and the holding part (4) designed as a lower-leg clasp or lower-leg strap (4.1) can be released by releasing the fastening elements (12, 12.1, 12.2, 12.3) and can be replaced by another clasp or strap (3.1, 4.1) which is adapted to the size of the upper and/or lower leg of a patient.

2. Modularly structured orthosis (1) according to claim 1,
**characterized in**
**that** the connecting element (5, 5.1, 5.2, 5.3) is designed as a one-part or multi-part hinge joint and/or the rail (6, 6.1, 6.2, 6.3) has a one-part or multi-part hinge joint.

3. Modularly structured orthosis (1) according to one of the previous claims, **characterized in**
**that** the connecting element (5, 5.1, 5.2, 5.3) has at least one receptacle (10, 10.1, 10.2, 10.3) for the rail (6, 6.1, 6.2, 6.3), the rail (6, 6.1, 6.2, 6.3) being connected to the holding part (3, 4) in a non-positive and/or positive manner via the receptacle (10, 10.1, 10.2, 10.3).

4. Modularly structured orthosis (1) according to claim 3,
**characterized in**
**that** the non-positive and/or positive connection between the holding part (3, 4) and the rail (6, 6.1, 6.2, 6.3) can be adjusted in height variably via the receptacle (10, 10.1, 10.2, 10.3) and/or the holding part (3, 4).

5. Modularly structured orthosis (1) according to one of the previous claims,
**characterized in**
**that** the connecting element (5, 5.1, 5.2, 5.3) comprises fastening elements (12, 12.2) for fastening strap receiving elements.

6. Modularly structured orthosis (1) according to one of the preceding claims, for insertion into a rail pocket of a bandage, wherein in particular a rail (6, 6.1, 6.2, 6.3) received in the rail pocket can be extended at the end.

7. Modularly structured orthosis (1) according to one of the previous claims,
**characterized in**
**that** the connecting element (5, 5.1, 5.2, 5.3) is designed symmetrically for the optional fastening of an upper or lower holding part (3, 4) and/or for the co-rotating and/or opposing contact of holding parts (3, 4) on the rail (6, 6.1, 6.2, 6.3).

8. Modularly structured orthosis (1) according to one of the previous claims,
**characterized in**
**that** the clasp (3.1, 4.1) is designed in one piece for insertion into the recesses (9, 9.1, 9.2, 9.3) of the connecting element (5, 5.1, 5.2, 5.3) and/or for passing through the recesses (9, 9.1, 9.2, 9.3) of the connecting element (5, 5.1, 5.2, 5.3).

9. Modularly structured orthosis (1) according to one of the previous claims,
**characterized in**
**that** fastening elements (12, 12.2) are designed on the rail (6, 6.1, 6.2, 6.3) for fastening receiving elements (7, 7.1, 7.2, 7.3, 7.4, 7.5, 7.6, 7.7, 7.8, 7.9, 7.10, 7.11), in particular strap receiving elements.

10. Modularly structured orthosis (1) according to one of the previous claims,
**characterized in**
**that** the rail (6, 6.1, 6.2, 6.3) has convex and/or concave regions which support a firm fit of the orthosis (1) on the patient.

11. Modularly structured orthosis (1) according to one of the previous claims,
**characterized in**
**that** the receiving element (7, 7.1, 7.2, 7.3, 7.4, 7.5, 7.6, 7.7, 7.8, 7.9, 7.10, 7.11) is connected to the connecting element (5, 5.1, 5.2, 5.3) and/or the rail (6, 6.1, 6.2, 6.3) in a non-positive and/or positive manner.

12. Modularly structured orthosis (1) according to one of the previous claims with one joint (2),
wherein the joint (2) connects at least two rails (6, 6.2 or 6.1, 6.3) in particular rails (6, 6.1, 6.2, 6.3) according to one of claims 1 to 4, and 6 to 7 and 10 to 12,
and via a connecting element (5, 5.1, 5.2, 5.3), in particular a connecting element (5, 5.1, 5.2, 5.3) according to one of the claims 1 to 5, 7 to 9 and 12, connects holding parts (3, 4), in particular holding parts (3, 4) according to one of the claims 1, 3, 4, 7 and 8, non-positively and/or positively connected to the rails (6, 6.1, 6.2, 6.3),
wherein two leg parts connected to the rails (6, 6.1, 6.2, 6.3), the leg ends (13, 13.1) of which facing one another are components of the rails (6, 6.1, 6.2, 6.3) and/or of the joint (2), are pivotably connected to one another via a bearing recess (14), preferably a guide slot and a joint insert (15) engaging the bearing recess (14).

13. Modularly structured orthosis (1) according to claim 12,
**characterized in**
**that** the joint insert (15) can be locked in different rotational positions on the associated rail (6, 6.1, 6.2, 6.3).

14. Modularly structured orthosis according to claim 12 or 13,
**characterized in**
**that** the joint insert (15) is designed as a circular disc (15.1) with a circumferential toothing, and in that the associated rail (6, 6.1, 6.2, 6.3) has a bearing recess (14) provided with an internal toothing corresponding to the circumferential toothing for receiving the joint insert.

15. Modularly structured orthosis (1) according to one of claims 12 to 14,
**characterized in**
**that** the bearing recess (14) provided for the joint insert (15, 15.1) forms, as a guide slot, stops which limit the mutual angle of rotation of the rails (6, 6.1, 6.2, 6.3).

## Revendications

1. Orthèse de construction modulaire (1), en particulier une orthèse de genou, comprenant les éléments suivants pouvant être assemblés mécaniquement :
- au moins une articulation de l'orthèse (2),
- une première pièce de maintien (3),
- une deuxième pièce de maintien (4),
- au moins un élément de liaison (5, 5.1, 5.2, 5.3),
- au moins un rail (6, 6.1, 6.2, 6.3),
les pièces de maintien (3, 4) peuvent être fixées chacune par un élément de liaison (5, 5.1, 5.2, 5.3) à ou sur un rail (6, 6.1, 6.2, 6.3), et au moins deux rails (6, 6.2 ou 6.1, 6.3) relient les première et deuxième pièces de maintien (3, 4) à l'articulation de l'orthèse (2), où la pièce de maintien est une agrafe (3.1, 4.1) ou une sangle,
dans laquelle la sangle et/ou l'agrafe (3.1, 4.1) peuvent être reliées au rail (6, 6.1, 6.2, 6.3) par l'intermédiaire de l'élément de liaison (5, 5.1, 5.2, 5.3), créant ainsi une orthèse de guidage de l'articulation sans ou avec limitation d'extension/flexion,
**caractérisé en ce que** des évidements (8, 8.1, 8.2, 8.3) sont formés dans l'agrafe et/ou la sangle (3.1, 4.1) pour le réglage et pour l'ajustement de la largeur de l'agrafe et/ou de la sangle (3.1, 4.1) au niveau des éléments de liaison (5, 5.1, 5.2, 5.3) ou dans ceux-ci, dans laquelle la pièce de maintien (3) et la pièce de maintien (4) sont fixées en tant qu'agrafe de cuisse ou sangle de cuisse (3.1) et en tant qu'agrafe de jambe ou sangle de jambe (4.1) dans des directions opposées l'une à l'autre, à savoir comme agrafe ou sangle avant (3.1) et comme agrafeou sangle arrière (4.1),
dans laquel l'élément de liaison (5, 5.1, 5.2, 5.3) comprend des éléments de fixation (12, 12.2) pour fixer des éléments récepteurs (7, 7.1, 7.2, 7.3, 7.4, 7.5, 7.6, 7.7, 7.8, 7.9, 7.10, 7.11)
dans laquelle tant la pièce de maintien (3) conçue comme une agrafe de cuisse ou une sangle de cuisse (3.1) que la pièce de maintien (4) conçue comme une agrafe de jambe ou une sangle de jambe (4.1) peuvent être libérées en relâchant les éléments de fixation (12, 12.1, 12.2, 12.3) et peuvent être remplacées par une autre agrafe ou sangle (3.1, 4.1) qui est adaptée à la taille de la jambe supérieure et/ou inférieure d'un patient.

2. Orthèse de construction modulaire (1) selon la revendication 1,
**caractérise en ce**
**que** l'élément de liaison (5, 5.1, 5.2, 5.3) est conçu comme une articulation à charnière en une ou plusieurs parties et/ou le rail (6, 6.1, 6.2, 6.3) a une articulation à charnière en une ou plusieurs parties.

3. Orthèse de construction modulaire (1) selon l'une des revendications précédentes, **caractérise en ce**
**que** l'élément de liaison (5, 5.1, 5.2, 5.3) présente au moins un logement (10, 10.1, 10.2, 10.3) pour le rail (6, 6.1, 6.2, 6.3), le rail (6, 6.1, 6.2, 6.3) étant relié à la pièce de maintien (3, 4) par l'intermédiaire du logement (10, 10.1, 10.2, 10.3) par adhérence et/ou par complémentarité de forme.

4. Orthèse de construction modulaire (1) selon la revendication 3,
**caractérise en ce**
**que** la liaison positive et/ou négative entre la pièce de maintien (3, 4) et le rail (6, 6.1, 6.2, 6.3) peut être réglée en hauteur de manière variable par le biais du logement (10, 10.1, 10.2, 10.3) et/ou de la pièce de maintien (3, 4).

5. Orthèse de construction modulaire (1) selon l'une des revendications précédentes,
**caractérise en ce**
**que** l'élément de liaison (5, 5.1, 5.2, 5.3) comprend des éléments de fixation (12, 12.2) pour la fixation des éléments récepteurs de la sangle.

6. Orthèse (1) de construction modulaire selon l'une des revendications précédentes, destinée à être insérée dans une poche de rail d'un bandage, dans laquelle en particulier un rail (6, 6.1, 6.2, 6.3) reçue dans la poche de rail peut être étendue à l'extrémité.

7. Orthèse de construction modulaire (1) selon l'une des revendications précédentes,
**caractérise en ce**
**que** l'élément de liaison (5, 5.1, 5.2, 5.3) est conçu symétriquement pour la fixation facultative d'une partie de retenue supérieure ou inférieure (3, 4) et/ou pour le contact co-rotatif et/ou opposé des pièces de maintien (3, 4) sur le rail (6, 6.1, 6.2, 6.3).

8. Orthèse de construction modulaire (1) selon l'une des revendications précédentes,
**caractérise en ce**
**que** l'agrafe (3.1, 4.1) est conçu en une seule pièce pour être inséré dans les évidements (9, 9.1, 9.2, 9.3) de l'élément de liaison (5, 5.1, 5.2, 5.3) et/ou pour passer à travers les évidements (9, 9.1, 9.2, 9.3) de l'élément de liaison (5, 5.1, 5.2, 5.3).

9. Orthèse de construction modulaire (1) selon l'une des revendications précédentes,
**caractérise en ce**
**que** les éléments de fixation (12, 12.2) sont conçus sur le rail (6, 6.1, 6.2, 6.3) pour fixer des éléments récepteurs (7, 7.1, 7.2, 7.3, 7.4, 7.5, 7.6, 7.7, 7.8, 7.9, 7.10, 7.11), en particulier des éléments récepteurs de sangle.

10. Orthèse de construction modulaire (1) selon l'une des revendications précédentes,
**caractérise en ce**
**que** le rail (6, 6.1, 6.2, 6.3) comporte des régions convexes et/ou concaves qui soutiennent un ajustement ferme de l'orthèse (1) sur le patient.

11. Orthèse de construction modulaire (1) selon l'une des revendications précédentes,
**caractérise en ce**
**que** l'élément récepteur (7, 7.1, 7.2, 7.3, 7.4, 7.5, 7.6, 7.7, 7.8, 7.9, 7.10, 7.11) est relié à l'élément de liaison (5, 5.1, 5.2, 5.3) et/ou au rail (6, 6.1, 6.2, 6.3) de manière non positive et/ou par complémentarité.

12. Orthèse de construction modulaire (1) selon l'une des revendications précédentes avec une articulation (2),
dans laquel l'articulation (2) relie au moins deux rails (6, 6.2 ou 6.1, 6.3) en particulier les rails (6, 6.1, 6.2, 6.3) selon l'une des revendications 1 à 4, et 6 à 7 et 10 à 12,
et par l'intermédiaire d'un élément de liaison (5, 5.1, 5.2, 5.3), en particulier un élément de liaison (5, 5.1, 5.2, 5.3) selon l'une des revendications 1 à 5, 7 à 9 et 12, relie des pièces de maintien (3, 4), en particulier des pièces de maintien (3, 4) selon l'une des revendications 1, 3, 4, 7 et 8, reliées aux rails (6, 6.1, 6.2, 6.3) par adhérence et/ou par complémentarité de forme,
dans laquel deux parties de jambe reliées aux rails (6, 6.1, 6.2, 6.3), dont les extrémités de jambe (13, 13.1) tournées l'une vers l'autre font partie des rails (6, 6.1, 6.2, 6.3) et/ou de l'articulation (2), sont reliées entre elles de manière pivotante par un évidement de palier (14), de préférence une fente de guidage et un insert d'articulation (15) s'engageant dans l'évidement de palier (14).

13. Orthèse de construction modulaire (1) selon la revendication 12,
**caractérise en ce**
**que** l'insert d'articulation (15) peut être verrouillé dans différentes positions de rotation sur le rail associé (6, 6.1, 6.2, 6.3).

14. Orthèse de construction modulaire selon la revendication 12 ou 13,
**caractérise en ce**
**que** l'insert d'articulation (15) est conçu comme un disque circulaire (15.1) avec une denture circonférentielle, et **en ce que** le rail associé (6, 6.1, 6.2, 6.3) présente un évidement de palier (14) pourvu d'une denture interne correspondant à la denture circonférentielle pour recevoir l'insert d'articulation.

15. Orthèse de construction modulaire (1) selon l'une des revendications 12 à 14,
**caractérise en ce**
**que** l'évidement de palier (14) prévu pour l'insert d'articulation (15, 15.1) forme, en tant que fente de guidage, des butées qui limitent l'angle de rotation mutuel des rails (6, 6.1, 6.2, 6.3).
